# EUROPEAN PATENT SPECIFICATION

(11) **EP 3 359 203 B1**
(45) Date of publication and mention of the grant of the patent: **31.07.2024**
(21) Application number: 16777683.0
(22) Date of filing: 05.10.2016
(51) Int. Cl.: A61K 51/08

(54) **RECEPTOR-BINDING DOMAINS LIGANDS FOR THE DETECTION, DIAGNOSIS AND TREATMENT OF PANCREATIC CANCER**
REZEPTORBINDENDE DOMÄNEN LIGANDEN ZUR DETEKTION, DIAGNOSE UND BEHANDLUNG VON PANKREASKREBS
LIGANDS DOMAINES DE LIAISON AU RÉCEPTEUR POUR LA DÉTECTION, LE DIAGNOSTIC ET LE TRAITEMENT DU CANCER DU PANCRÉAS

(30) Priority: 05.10.2015 EP 15188435
(43) Date of publication of application: 15.08.2018
(73) Proprietor: Metafora Biosystems, 75014 Paris (FR); Centre National de la Recherche Scientifique, 75016 Paris (FR); Université de Montpellier, 34090 Montpellier (FR); INSERM - Institut National de la Santé et de la Recherche Médicale, 75654 Paris Cedex 13 (FR)
(72) Inventor: TOUHAMI, Jawida, 34070 Montpellier (FR); E, Guangqi, 75012 Paris (FR); GIOVANNINI, Donatella, 34000 Montpellier (FR); ROBERT, Bruno, 34270 Valflaunès (FR); SEVESTRE, Samuel, 34970 Lattes (FR); BUSSON, Muriel, 34000 Montpellier (FR); BATTINI, Jean-Luc, 34090 Montpellier (FR); PETIT, Vincent, 75014 Paris (FR); SITBON, Marc, 34000 Montpellier (FR)
(74) Representative: Icosa
(86) International application number: PCT/EP2016/073797
(87) International publication number: WO 2017/060304

(56) References cited:
- WO-A1-2010/079208
- WO-A1-2012/035166
- WO-A1-2013/060893
- US-B1- 7 473 531

## Description

### FIELD OF INVENTION

The present invention relates to the field of pancreatic cancer management, in particular to method for diagnosing and treating pancreatic cancer. In particular, the present invention relates to receptor-binding domains (RBD) ligands for the treatment and diagnosis of pancreatic tumors, especially for use as a probe for medical imaging.

### BACKGROUND OF INVENTION

Pancreatic cancer is the fourth most common cause of cancer-related death in the western world. Pancreatic cancer arises when cells in the pancreas, a glandular organ behind the stomach, begin to multiply out of control and form a mass. These cancer cells have the ability to invade other parts of the body. There are a number of types of pancreatic cancer. Pancreatic cancer is extremely aggressive and, at the time of diagnosis, less than 20% of patients present with a sufficiently localized tumor to allow curative treatment.

Cancer in general can be diagnosed using several technics and among them are the medical imaging technics, such as computed tomography (CT scan) and endoscopic ultrasound (EUS). Magnetic resonance imaging (MRI) and positron emission tomography (PET) may also be used, and magnetic resonance cholangiopancreatography may be useful in some cases. However, such technics require the finding of specific biomarkers. One of the most renowned biomarkers of cancer is GLUT1. Therefore, it is well recognized to use a radioactive glucose homologue, 18F-fluoro-2-deoxyglucose (FDG), for PET imaging since increase of glucose transfer and glycolytic activities are hallmarks of a majority of cancer cells. However, some cancers such as pancreatic cancer, do not reliably exhibit overexpression of GLUT 1.

US7473531B1 discloses a method for diagnosing pancreatic cancer in an individual, comprising detecting a higher level of ASCT2 protein in a sample from the individual as compared to levels of ASCT2 protein in pancreatic tissues of healthy individuals. As detection means, monoclonal antibodies directed against ASCT2 are used.

So far, no specific biomarker (and in particular no metabolic biomarker) of pancreatic cancers or cell lines derived from pancreatic cancer tumors has been found. Therefore there is an urgent need to find such biomarkers that allow an accurate and definitive detection of pancreatic cancer so as to be able to define a therapeutic strategy right from the early stages of the disease.

The applicants surprisingly found that peculiar RBD ligands specifically recognize pancreatic tumor cell lines. In addition, the Applicants discovered that a RBD ligand could recognize specifically all model pancreatic tumor cells *in vivo* using medical imaging in mammals.

### SUMMARY

The present invention relates to a ligand comprising a part or the totality of a receptor-binding domain (RBD) derived from the soluble part of a glycoprotein of an enveloped virus for use for the *in vivo* detection of pancreatic cancer cells in a patient, wherein said ligand is specific of a cell surface nutrient transporter selected from ASCT1, ASCT2 and/or XPR1.In one embodiment, said means are for the diagnosis or monitoring of pancreatic cancer.

In another embodiment, said means are for the diagnosis or monitoring of pancreatic cancer preferably by medical imaging, preferably by magnetic resonance imaging (MRI), X-ray-based imaging techniques such as computed tomography (CT), radiography, positron-emission tomography (PET), single photon emission tomography (SPECT), endoscopic ultrasound (EUS), magnetic resonance cholangiopancreatography, fluorimetry, fluoroscopy, fluorescence, and near-infrared (NIR) fluorescent imaging.

In another embodiment, said mean is a RBD ligand selected from the group comprising HERV-W.RBD, RD114.RBD, BaEV.RBD, SNV.RBD, SRV.RBD, MPMV.RBD, Xeno.RBD, variants and fragments thereof. Preferably, said mean is a RBD ligand selected from the group comprising HERV-W.RBD, RD114.RBD, Xeno.RBD, variants and fragments thereof.

In another embodiment, said RBD ligand is coupled with at least one contrast agent, wherein said contrast agent is preferably selected from a radiolabeled agent or a fluorescent agent.

The present disclosure also relates to a receptor-binding domain (RBD) ligand coupled with at least one contrast agent, which is not covered by the claimed invention, wherein said RBD is selected from the group HERV-W.RBD, RD114.RBD, BaEV.RBD, SNV.RBD, SRV.RBD, MPMV.RBD, Xeno.RBD, variants and fragments thereof, preferably from HERV-W.RBD, RD114.RBD, Xeno.RBD, variants and fragments thereof.

In one embodiment not covered by the claimed invention, said RBD ligand is for use as a probe for medical imaging.

The present invention also relates to an *in vitro* method for detecting pancreatic cancer cells, wherein said method comprises measuring the expression level of at least one cell surface nutrient transporter selected from ASCT1, ASCT2 and/or XPR1.

In another embodiment, said *in vitro* method further comprises comparing the measured expression level with a reference expression level.

In another embodiment, said *in vitro* method is for diagnosing or monitoring pancreatic cancer in a subject.

In another embodiment, said expression level is assessed at the protein level, preferably the measurement of the expression level of at least one cell surface nutrient transporter corresponds to the detection and quantification of said at least one cell surface nutrient transporter on the cell surface, more preferably by detecting and/or quantifying binding of a ligand to a cell surface nutrient transporter, wherein preferably, said ligand is an antibody or is a receptor-binding domain ligand (RBD) comprising a part or the totality of a receptor-binding domain (RBD) derived from the soluble part of a glycoprotein of an enveloped virus.

In another embodiment, said RBD ligand is HERV-W.RBD, RD114.RBD, BaEV.RBD, SNV.RBD, SRV.RBD, MPMV.RBD, or Xeno.RBD, variants or fragments thereof. Preferably said RBD ligand is selected from HERV-W.RBD, RD114.RBD and Xeno.RBD, variants and fragments thereof.

The present invention also relates to a diagnostic composition comprising at least one RBD ligand for use as described hereinabove, or at least one RBD ligand as described hereinabove, and a pharmaceutically acceptable excipient.

The present invention also relates to a kit for the diagnosis of a pancreatic cancer, comprising at least one RBD ligand as described hereinabove, or at least one RBD ligand as described hereinabove, or a diagnostic composition as described hereinabove.

The present invention also relates to a pharmaceutical composition for use for the treatment of pancreatic cancer comprising at least one RBD ligand selected from the group comprising HERV-W.RBD, RD114.RBD, BaEV.RBD, SNV.RBD, SRV.RBD, MPMV.RBD, Xeno.RBD, variants and fragments thereof, and a pharmaceutically acceptable excipient. Preferably said RBD ligand is selected from HERV-W.RBD, RD114.RBD, and Xeno.RBD, variants and fragments thereof.

### DEFINITIONS

In the present invention, the following terms have the following meanings:
- The term **"diagnostic composition"** refers to a composition to be administered in a subject in order to perform a diagnosis and in particular an *in vivo* diagnosis. In the present invention, a diagnostic composition is for detecting pancreatic cancer cells, preferably within the body of a subject.
- The term **"effective amount"** refers to the level or amount of a ligand, preferably of a RBD ligand that is aimed at, without causing significant negative or adverse side effects to the target, binding to a cell surface receptor, preferably a cell surface nutrient transporter.
- The term **"therapeutically effective amount"** means level or amount of agent that is aimed at, without causing significant negative or adverse side effects to the target, (1) delaying or preventing the onset of a pancreatic cancer; (2) slowing down or stopping the progression, aggravation, or deterioration of one or more symptoms of pancreatic cancer; (3) bringing about ameliorations of the symptoms of pancreatic cancer; (4) reducing the severity or incidence of pancreatic cancer; or (5) curing pancreatic cancer. A therapeutically effective amount may be administered prior to the onset of pancreatic cancer, for a prophylactic or preventive action. Alternatively or additionally, the therapeutically effective amount may be administered after initiation of pancreatic cancer, for a therapeutic action.
- The term **"treatment"** refers to both therapeutic treatment and prophylactic or preventative measures; wherein the object is to prevent or slow down (lessen) pancreatic cancer. Those in need of treatment include those already with pancreatic cancer as well as those prone to have pancreatic cancer or those in whom pancreatic cancer is to be prevented. A subject or mammal is successfully "treated" for a disease if, after receiving a therapeutic amount of an anti-cancer agent, the patient shows observable and/or measurable reduction in or absence of one or more of the following: reduction in the number of pathogenic cells; reduction in the percent of total cells that are pathogenic; and/or relief to some extent, of one or more of the symptoms associated with the specific disease or condition; reduced morbidity and mortality, and improvement in quality of life issues. The above parameters for assessing successful treatment and improvement in the disease are readily measurable by routine procedures familiar to a physician.
- The term **"identity",** when used in a relationship between the sequences of two or more polypeptides or of two or more DNA sequences, refers to the degree of sequence relatedness between polypeptides or DNA sequences (respectively), as determined by the number of matches between strings of two or more amino acid residues or of two or more nucleotides, respectively. "Identity" measures the percent of identical matches between the smaller of two or more sequences with gap alignments (if any) addressed by a particular mathematical model or computer program (i.e., "algorithms"). Identity of related polypeptides or DNA sequences can be readily calculated by known methods. Such methods include, but are not limited to, those described in Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part 1, Griffin, A. M., and Griffin, H. G., eds., Humana Press, New Jersey, 1994; Sequence Analysis in Molecular Biology, von Heinje, G., Academic Press, 1987; Sequence Analysis Primer, Gribskov, M. and Devereux, J., eds., M. Stockton Press, New York, 1991; and Carillo et al., SIAM J. Applied Math. 48, 1073 (1988). Preferred methods for determining identity are designed to give the largest match between the sequences tested. Methods of determining identity are described in publicly available computer programs. Preferred computer program methods for determining identity between two sequences include the GCG program package, including GAP (Devereux et al., Nucl. Acid. Res. \2, 387 (1984); Genetics Computer Group, University of Wisconsin, Madison, Wis.), BLASTP, BLASTN, TBLASTN and FASTA (Altschul et al., J. Mol. Biol. 215, 403-410 (1990)). The BLASTX program is publicly available from the National Center for Biotechnology Information (NCBI) and other sources (BLAST Manual, Altschul et al. NCB/NLM/NIH Bethesda, Md. 20894; Altschul *et al.,* supra). The well-known Smith Waterman algorithm may also be used to determine identity.
- As used herein, the term **"pharmaceutically acceptable excipient"** refers to an excipient that does not produce an adverse, allergic or other untoward reaction when administered to an animal, preferably a human. It includes any and all solvents, dispersion media, coatings, antibacterial and antifungal agents, isotonic and absorption delaying agents and the like. For human administration, preparations should meet sterility, pyrogenicity, general safety and purity standards as required by regulatory offices, such as, for example, FDA Office or EMA.
- The term **"subject"** refers to a mammal, preferably a human. In one embodiment, a subject may be a "patient", i.e. a warm-blooded animal, more preferably a human, who/which is awaiting the receipt of, or is receiving medical care or was/is/will be the object of a medical procedure, or is monitored for the development of a disease.
- **"XPR1"** refers to a phosphate exporter expressed by metazoans, in particular by humans, used as receptor by xenotropic murine leukemia virus (MLV), polytropic MLV and xenotropic murine leukemia virus-related virus (XMRV) (Giovannini et al., Cell Reports 3, 1866-1873, 2013). In one embodiment, XPR1 is human XPR1 (accession number AAH41142, SEQ ID NO: 21) encoded by SEQ ID NO: 22 (accession number BC041142.1). In one embodiment, XPR1 comprises or consists of an amino acid sequence presenting a sequence identity of at least 70% with SEQ ID NO: 21, preferably a sequence identity of at least 75, 80, 85, 90, 91, 92, 93, 94, 95, 96, 97, 98, 99% or more with SEQ ID NO: 21. In one embodiment, XPR1 is encoded by a nucleotide sequence presenting a sequence identity of at least 70% with SEQ ID NO: 22, preferably a sequence identity of at least 75, 80, 85, 90, 91, 92, 93, 94, 95, 96, 97, 98, 99% or more with SEQ ID NO: 22. In one embodiment, XPR1 comprises or consists of a fragment of SEQ ID NO: 21, preferably a fragment of at least about 100 amino acids, more preferably of at least about 150, 200, 250, 300, 350, 400, 450, 500, 550 or 600 amino acids.
- **"ASCT1"** refers to a glutamate and neutral amino acid transporter. ASCT1 is used as receptor for human endogenous retrovirus W (HERV-W), RD 114 feline gammaretrovirus, baboon endogenous virus (BaEV), spleen necrosis virus (SNV), simian retrovirus (SRV) and Mason-Pfizer monkey virus (MPMV). In one embodiment, ASCT1 is human ASCT1 (accession number AAH26216.1, SEQ ID NO: 19) encoded by SEQ ID NO: 20 (accession number HUMASCT1A). In one embodiment, ASCT1 comprises or consists of an amino acid sequence presenting a sequence identity of at least 70% with SEQ ID NO: 19, preferably a sequence identity of at least 75, 80, 85, 90, 91, 92, 93, 94, 95, 96, 97, 98, 99% or more with SEQ ID NO: 19. In one embodiment, ASCT1 is encoded by a nucleotide sequence presenting a sequence identity of at least 70% with SEQ ID NO: 20, preferably a sequence identity of at least 75, 80, 85, 90, 91, 92, 93, 94, 95, 96, 97, 98, 99% or more with SEQ ID NO: 20. In one embodiment, ASCT1 comprises or consists of a fragment of SEQ ID NO: 19, preferably a fragment of at least about 100 amino acids, more preferably of at least about 150, 200, 250, 300, 350, 400, 450, 500 amino acids.
- **"ASCT2"** refers to a glutamine and other neutral amino acid transporter. ASCT2 is used as receptor for human endogenous retrovirus W (HERV-W), RD 114 feline gammaretrovirus, baboon endogenous virus (BaEV), spleen necrosis virus (SNV), simian retrovirus (SRV) and Mason-Pfizer monkey virus (MPMV). In one embodiment, ASCT2 is human ASCT2 (accession number Q15758.2, SEQ ID NO: 33) encoded by SEQ ID NO: 34 (accession number GQ919058). In one embodiment, ASCT2 comprises or consists of an amino acid sequence presenting a sequence identity of at least 70% with SEQ ID NO: 33, preferably a sequence identity of at least 75, 80, 85, 90, 91, 92, 93, 94, 95, 96, 97, 98, 99% or more with SEQ ID NO: 33. In one embodiment, ASCT2 comprises or consists of a fragment of SEQ ID NO: 33, preferably a fragment of at least about 100 amino acids, more preferably of at least about 150, 200, 250, 300, 350, 400, 450, 500 amino acids. In one embodiment, ASCT2 is encoded by a nucleotide sequence presenting a sequence identity of at least 70% with SEQ ID NO: 34, preferably a sequence identity of at least 75, 80, 85, 90, 91, 92, 93, 94, 95, 96, 97, 98, 99% or more with SEQ ID NO: 34.
- **"About"** preceding a figure means plus or less 10% of the value of said figure.

### DETAILED DESCRIPTION

The present invention relates to a ligand comprising a part or the totality of a receptor-binding domain (RBD) derived from the soluble part of a glycoprotein of an enveloped virus for use in a method for the *in vivo* detection of pancreatic cancer cells in a patient, wherein said ligand is specific of a cell surface nutrient transporter selected from ASCT1, ASCT2 and/or XPR1.

The present invention further relates to an *in vitro* method for detecting a pancreatic cancer cell, wherein said method comprises determining or measuring the expression level of at least one cell surface nutrient transporter, wherein said at least one cell surface nutrient transporter comprises or consists in ASCT1 and/or ASCT2 and/or XPR1, wherein said expression level is assessed at the protein level, by detecting and/or quantifying binding of a ligand to a cell surface nutrient transporter, wherein said ligand comprises a part or the totality of a RBD derived from the soluble part of a glycoprotein of an enveloped virus.

In one embodiment, said method further comprises comparing the measured expression level with a reference expression level.

In one embodiment, the method of the invention comprises measuring the expression of ASCT1. In another embodiment, the method of the invention comprises measuring the expression of ASCT2. In another embodiment, the method of the invention comprises measuring the expression of ASCT1 and ASCT2. In another embodiment, the method of the invention comprises measuring the expression of XPR1. In another embodiment, the method of the invention comprises measuring the expression of XPR1 and ASCT1, or of XPR1 and ASCT2. In another embodiment, the method of the invention comprises measuring the expression of XPR1, ASCT1 and ASCT2.

In one embodiment, the method of the invention is an *in vitro* or *ex vivo* method, i.e. the method of the invention is performed on a cell sample, such as, for example, a cell line or a sample of cells previously recovered from a subject (respectively).

In another embodiment not covered by the claimed invention, the method is an *in vivo* method, i.e. the method is for detecting pancreatic cancer cells directly within the body of the subject, such as, for example, by medical imaging.

As used herein, the term "cell surface nutrient transporter" refers to a nutrient transporter anchored in the plasma membrane of a cell. Mammalian cells take up necessary nutrients via "nutrient transporters" on the cell surface and expel catabolites and other components. Nutrients and metabolites or catabolites are, for example, carbohydrates, amino acids, inorganic phosphate, nucleosides, lipids, vitamins, heme, ions, etc. Nutrient transporters may be divided based on passive or active mechanisms of function. Passive (or facilitated) transporters allow diffusion of solutes across membranes down their electrochemical gradient. Active transporters create solute gradients across membranes, utilizing diverse energy-coupling mechanisms, such as, for example, ATP synthesis or hydrolysis. In one embodiment, the cell surface nutrient transporter belongs to the SLC series, wherein SLC stands for Solute Linked Carriers or Solute Carriers.

Examples of cell surface nutrient transporters include, but are not limited to, transporters of glucose, such as, for example, glucose importers (such as, for example, GLUT1); transporters of inorganic phosphate, such as, for example, inorganic phosphate importers (such as, for example, PiT1 or PiT2) or inorganic phosphate exporters (such as, for example, XPR1); transporters of amino acids, such as, for example, transporters of neutral amino acids (such as, for example, neutral amino acids importers (such as, for example, ASCT1 or ASCT2)), or transporters of cationic amino acids (such as, for example, CAT1); transporters of heme (such as, for example, FLVCR1); transporters of inositol, such as, for example, transporters of myo-inositol (such as, for example, SMIT1); and transporters of riboflavin, such as, for example, importers of riboflavin (such as, for example, RFT1, RFT3, PARI or PAR2).

In one embodiment, the cell surface nutrient transporter is a transporter of neutral amino acids, such as, for example, neutral amino acids importers (such as, for example, ASCT1 and/or ASCT2) or a transporter of inorganic phosphate, such as, for example, an inorganic phosphate exporter (such as, for example, XPR1).

As used herein, the term "expression" may refer alternatively to the transcription of a cell surface receptor, preferably a cell surface nutrient transporter (i.e. expression of the RNA) or to the translation (i.e. expression of the protein) of a cell surface receptor, preferably a cell surface nutrient transporter, or to the presence of the cell surface receptor, preferably a cell surface nutrient transporter at the surface of the cell.

Methods for determining the expression level are well-known from the skilled artisan, and include, without limitation, determining the transcriptome (in an embodiment wherein expression relates to transcription of a receptor, preferably a cell surface nutrient transporter) or proteome (in an embodiment wherein expression relates to translation of a receptor, preferably cell surface nutrient transporter) of a cell.

In one embodiment of the invention, the expression of the cell surface receptor, preferably cell surface nutrient transporter is assessed at the RNA level.

Methods for assessing the transcription level of a transporter are well known in the prior art. Examples of such methods include, but are not limited to, RT-PCR, RT-qPCR, Northern Blot, hybridization techniques such as, for example, use of microarrays, and combination thereof including but not limited to, hybridization of amplicons obtained by RT-PCR, sequencing such as, for example, next-generation DNA sequencing (NGS) or RNA-seq (also known as "Whole Transcriptome Shotgun Sequencing") and the like.

Examples of PCR or qPCR primers that may be used for assessing the expression of XPR1 include, but are not limited to, the following couple of primers: Forward primer: 5'-AGAGCTTGGGAGACAAAGCA-3' (SEQ ID NO: 23) - Reverse primer: 5'-GTGGACACAACATTCGCAAC-3' (SEQ ID NO: 24).

Examples of PCR or qPCR primers that may be used for assessing the expression of ASCT2 include, but are not limited to, the following couple of primers: Forward primer: 5'-ATCGTGGAGATGGAGGA-3' (SEQ ID NO: 25) - Reverse primer: 5'-AAGAGGTCCCAAAGGCAG-3' (SEQ ID NO: 26).

In one embodiment of the invention, the expression of the cell surface receptor, preferably cell surface nutrient transporter is assessed at the protein level.

*In vitro* methods for determining a protein level in a sample are well-known in the art. Examples of such methods include, but are not limited to, immunohistochemistry, Multiplex methods (Luminex), Western blot, enzyme-linked immunosorbent assay (ELISA), sandwich ELISA, fluorescent-linked immunosorbent assay (FLISA), enzyme immunoassay (EIA), radioimmunoassay (RIA), flow cytometry (FACS) and the like.

*In vivo* methods for determining a protein level are well-known in the art. Examples of such methods include, but are not limited to, computed tomography (CT scan), endoscopic ultrasound (EUS), magnetic resonance imaging (MRI), positron-emission tomography (PET), single photon emission tomography (SPECT), magnetic resonance cholangiopancreatography, fluorimetry, fluorescence, and near-infrared (NIR) fluorescent imaging.

In one embodiment of the invention, determining the expression level of a cell surface receptor, preferably cell surface nutrient transporter, specifically corresponds to the detection and quantification of said receptor, preferably cell surface nutrient transporter, present on the cell surface.

Methods for analyzing the presence of a protein on the cell surface are well-known to the skilled artisan and include, without limitation, FACS analysis, immunohistochemistry, western blot associated with cell fractionation, enzyme-linked immunosorbent assay (ELISA), sandwich ELISA, fluorescent-linked immunosorbent assay (FLISA), enzyme immunoassay (EIA), radioimmunoassay (RIA), image analysis, for example high content analysis, computed tomography (CT scan), endoscopic ultrasound (EUS), magnetic resonance imaging (MRI), positron-emission tomography (PET), single photon emission tomography (SPECT), magnetic resonance cholangiopancreatography, fluorimetry, fluorescence, and near-infrared (NIR) fluorescent imaging and the like.

In one embodiment, determining the expression level of at least one cell surface receptor, preferably at least one cell surface nutrient transporter corresponds to detecting and/or quantifying binding of a ligand to a cell surface receptor, preferably to a cell surface nutrient transporter.

As used herein, the term "ligand" refers to any substance that forms a complex with a cell surface receptor, preferably with a cell surface nutrient transporter. Typical ligands include, but are not limited to, polypeptides and proteins. As used herein, a polypeptide refers to a linear polymer of amino acids (preferably at least 50 amino acids) linked together by peptide bonds. A protein specifically refers to a functional entity formed of one or more polypeptides, and optionally of non-polypeptides cofactors.

Preferably, said ligand is a receptor-binding domain ligand and the method of the invention comprises detecting and/or quantifying a complex formed between said receptor-binding domain ligand and a cell surface receptor, preferably a cell surface nutrient transporter. In another embodiment, said ligand is an antibody specific of said cell surface receptor, and the method of the invention comprises detecting and/or quantifying a complex formed between said antibody and said cell surface receptor.

The expression "detecting and/or quantifying binding of a ligand, such as, for example, a receptor-binding domain ligand, to a cell surface receptor, preferably to a cell surface nutrient transporter" means that when a cell surface receptor, preferably a cell surface nutrient transporter is present, a complex is formed between the receptor, preferably the cell surface nutrient transporter and the ligand.

In one embodiment, that complex can be detected if the ligand has been, for example, but not limited to, covalently coupled with a detectable molecule such as an antibody constant fragment (Fc) or a fluorescent compound (e.g. Cyanine dye, Alexa dye, Quantum dye, etc). The complex can also be detected if the ligand has been tagged with different means well known to the person skilled in the art. For example, but without limitation, a tag used in the invention can be a tag selected from the group comprising or consisting of Hemagglutinin Tag, Poly Arginine Tag, Poly Histidine Tag, Myc Tag, Strep Tag, S-Tag, HAT Tag, 3x Flag Tag, Calmodulin-binding peptide Tag, SBP Tag, Chitin binding domain Tag, GST Tag, Maltose-Binding protein Tag, Fluorescent Protein Tag, T7 Tag, V5 Tag and Xpress Tag. The use of the ligand therefore allows on the one hand the identification and detection of the cell surface receptor, preferably of the cell surface nutrient transporter depending on the ligand used, and on the other hand the quantification of the complex formed. In one embodiment, detecting or quantifying binding is conducted by flow cytometry, immunofluorescence or image analysis, for example, high content analysis.

In another embodiment, that complex can be detected if the ligand has been for example, but not limited to, covalently coupled with at least one contrast agent. In one embodiment, detecting or quantifying binding is conducted by medical imaging technics.

As used herein, the term "contrast agent" refers to agents used to improve the visibility of internal bodily structures in medical imaging technics, including, but not limited to, magnetic resonance imaging (MRI), X-ray-based imaging techniques such as computed tomography (CT), radiography, endoscopic ultrasound (EUS), positron-emission tomography (PET), single photon emission tomography (SPECT), magnetic resonance cholangiopancreatography, fluoroscopy, fluorimetry, fluorescence, and near-infrared (NIR) fluorescent imaging.

In one embodiment, the ligand is coupled with at one least contrast agent, wherein said contrast agent may be a radiolabeled agent or a fluorescent agent.

In one embodiment not covered by the claimed invention, the radiolabeled agent is selected from the group comprising a non-metallic radioisotope, non-metallic or metallic dye, paramagnetic metal, or radioactive metal.

Examples of non-metallic radioisotopes comprise but are not limited to: I-125, I-123, I-131, C-11, F-18, Br-75, Br-76, Br-77, Br-80, and At-211. The non-metallic radioisotopes may be conjugated covalently to either terminus of the ligand, functional groups of amino acid side chains, be part of a linear stabilized peptide as an additional substituent, e.g. in an amino acid phenylalanine or tyrosine carrying fluorine, bromine or iodine, or as an additional substituent carboxy or methyl, or as a replacement of any regular carbon atom in the ligand by I-125. Preferably, the ligand is coupled with I-125. These radioisotopes are useful in ligands as positron emission tomography (PET) probes or as single-photon emission computed tomography (SPECT) probes.

Examples of non-metallic or metallic dyes comprise, but are not limited to, organic molecules, e.g., commercial Alexa fluor dyes, fluorescein, rhodamine, or Cy5.5, complexes of transition metals, e.g. chelates of Eu³⁺, Tb³⁺, or nanoparticles (quantum dots) which adsorb and/or emit light in the visible range or in the near infrared. Organic dyes and chelating systems will be coupled to the ligands as described above for chelators. Conjugation of the ligands with quantum dots is done by procedures known to those skilled in the art. These ligands carrying dyes are useful as optical imaging probes.

Examples of paramagnetic metals comprise, but are not limited to, Gd, Fe, Mn. The metals are attached to the ligands. These ligands are useful as magnetic resonance imaging (MRI) probes.

Examples of radioactive metals comprise but are not limited to: Tc-99m, Ga-67, Ga-68, Lu-177, Cu-64, and Zr-89, Re-186/188, Bi-213, Y- 90, Cu-67, Lu-177, Tb-161, Tc-99m, and In-111. The radioactive metals (and the paramagnetic metals mentioned above) are attached to the ligands of the invention through chelators as listed above, directly connected to the ligands or through a spacer.

Examples of fluorescent agents include, but are not limited to, GFP, mPlum^{g}, mCherry^{g}, tdTomato^{g}, mStrawberry^{g}, J-Red, DS-Red monomer^{h}, mOrange^{g}, mKO, mCitrineⁱ, Venus, YPet^{g}, EYFP, Emerald^{g}, EGFP, CyPet, mCFP^{m}, Cerulean^{g}, T-Sapphire^{g}, indocyanine green, ZW800-1, Cy5.5 and IRDye800CW.

In one embodiment, the contrast agent is I-125.

In one aspect of the invention, the ligand is a RBD ligand, wherein said RBD ligand comprises a part or the totality of a receptor-binding domain (RBD) derived from the soluble part of a glycoprotein of an enveloped virus that interacts with a cell surface receptor. Examples of such receptors include, but are not limited to, a nutrient transporter, an integral protein, a GPI-anchored protein, a polysaccharide, a hetero- or proteoglycan or any other component of the extracellular matrix, all examples shown to be viral receptors. In one embodiment, the ligand is soluble, i.e. it does not comprise a transmembrane domain, and is therefore not anchored to a membrane.

The expression "derived from the soluble part of the glycoprotein of an enveloped virus" means that the ligand is a fragment or a part of a glycoprotein contained in the envelope of a virus and can be obtained, for example, by cloning.

The term "glycoprotein" is to be understood as meaning an envelope glycoprotein, a coat glycoprotein or a fusion glycoprotein, wherein the term "glycoprotein" refers to a protein containing oligosaccharide chains covalently attached to polypeptide side-chains.

The expression "that interacts with a cell surface receptor" means that the glycoprotein is liable to recognize a receptor present on the surface of the cell. In one embodiment, a ligand that interacts with a cell surface receptor, preferably with a cell surface nutrient transporter, will thus form a complex with said cell surface receptor, which complex may be detected by a method as here above described.

RBDs may be found, in particular, in glycoproteins of the envelope of viruses.

In one embodiment, the receptor-binding domain ligand contains the total RBD or a fragment or part of the RBD.

In one embodiment, the RBD ligand of the invention is glycosylated. In another embodiment, the RBD ligand of the invention is not glycosylated.

In one embodiment, the ligand of the invention comprises the SU domain of the glycoprotein envelope of a virus or a fragment of the SU domain, such as, for example, the RBD. In another embodiment, the ligand of the invention does not comprise the TM domain of the glycoprotein envelope of a virus. Therefore, in one embodiment of the invention, the ligand of the invention is a soluble peptide, such as, for example, a soluble RBD. As used herein, the term "soluble peptide" refers to a peptide which is not anchored within a membrane, such as, for example, by a transmembrane or a GPI anchor domain.

In one embodiment, said virus is selected from the group comprising retroviruses, such as, for example, (i) gammaretroviruses such as for example, murine (MLV), feline (FeLV, RD114), avian (spleen necrosis virus (SNV), or simian baboon endogenous virus (BaEV), simian sarcoma virus (SSV), SSV-associated virus (SSaV) or betaretroviruses known to have (glyco)protein envelopes related to that of gammaretroviruses such as for example simian retrovirus (SRV) and Mason-Pfizer monkey virus (MPMV); and (ii) deltaretroviruses such as, for example, primate T cell leukaemia virus (such as, for example, human T cell leukaemia virus (HTLV) and simian T cell leukaemia virus (STLV)) and bovine leukemia virus (BLV).

The gamma and deltaretroviruses encode an Env glycoprotein present in mature retrovirus virions. The Env protein is synthesized in the form of a propeptide, which is dived in Golgi apparatus by furine peptidase, resulting in two polypeptides: the transmembrane (TM) and the cell surface (SU) components. The SU domain contains two major subdomains: a domain of interaction with the TM domain and the RBD, the further being liable to interact with host cell membrane receptors.

In one embodiment of the invention, the RBD ligand is isolated from the glycoprotein of human endogenous retrovirus W, and is herein referred as HERV-W.RBD. In one embodiment, the receptor-binding domain ligand comprises a part or the totality of HERV-W.RBD and binds to the ASCT1 and/or ASCT2 nutrient transporter(s).

In one embodiment, said HERV-W.RBD comprises or consists of the amino acid sequence SEQ ID NO: 1, variants or fragments thereof.

In one embodiment, said fragment comprises or consists of amino acids 21 to 189 of SEQ ID NO: 1.

In one embodiment, said fragment comprises or consists of amino acids 1 to 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 or 188 of SEQ ID NO: 1.

In another embodiment, said fragment comprises or consists of amino acids 21 to 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 or 188 of SEQ ID NO: 1.

In one embodiment, said HERV-W.RBD comprises or consists of the amino acid sequence SEQ ID NO: 46, variants or fragments thereof.

In one embodiment, said fragment comprises or consists of amino acids 21 to 189 of SEQ ID NO: 46.

In one embodiment, said fragment comprises or consists of amino acids 1 to 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 or 188 of SEQ ID NO: 46.

In another embodiment, said fragment comprises or consists of amino acids 21 to 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187 or 188 of SEQ ID NO: 46.

In another embodiment, said fragment comprises or consists of SEQ ID NO: 2, encoded by the DNA sequence SEQ ID NO: 3.

In another embodiment, said fragment comprises or consists of amino acids 21 to 121 of SEQ ID NO: 2.

In one embodiment, said HERV-W.RBD comprises or consists of the amino acid sequence SEQ ID NO: 38, variants or fragments thereof.

In one embodiment, said fragment comprises or consists of amino acids 22 to 181 of SEQ ID NO: 38.

In one embodiment, said fragment comprises or consists of amino acids 1 to 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, or 180 of SEQ ID NO: 38.

In one embodiment, said fragment comprises or consists of amino acids 22 to 130, 131, 132, 133, 134, 135, 136, 137, 138, 139, 140, 141, 142, 143, 144, 145, 146, 147, 148, 149, 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, or 180 of SEQ ID NO: 38.

In one embodiment of the invention, the RBD ligand is isolated from the glycoprotein of xenotropic murine leukemia virus, and is herein referred as Xeno.RBD. In one embodiment, the receptor-binding domain ligand comprises a part or the totality of Xeno.RBD and binds to the XPR1 nutrient transporter.

In one embodiment, said Xeno.RBD comprises or consists of the amino acid sequence SEQ ID NO: 4 or fragments thereof.

In one embodiment, said fragment comprises or consists of amino acids 36 to 316 of SEQ ID NO: 4.

In one embodiment, said fragment comprises or consists of amino acids 1 to 297, 298, 299, 300, 301, 302, 303, 304, 305, 306, 307, 308, 309, 310, 311, 312, 313, 314 or 315 of SEQ ID NO: 4.

In another embodiment, said fragment comprises or consists of amino acids 34 to 297, 298, 299, 300, 301, 302, 303, 304, 305, 306, 307, 308, 309, 310, 311, 312, 313, 314 or 315 of SEQ ID NO: 4.

In another embodiment, said fragment comprises or consists of amino acids 36 to 297, 298, 299, 300, 301, 302, 303, 304, 305, 306, 307, 308, 309, 310, 311, 312, 313, 314 or 315 of SEQ ID NO: 4.

In another embodiment, said fragment comprises or consists of SEQ ID NO: 5.

In another embodiment, said fragment comprises or consists of amino acids 34 to 296 of SEQ ID NO: 5.

In another embodiment, said fragment comprises or consists of amino acids 36 to 296 of SEQ ID NO: 5.

In another embodiment, said fragment comprises or consists of SEQ ID NO: 47, encoded by the DNA sequence SEQ ID NO: 6.

In another embodiment, said fragment comprises or consists of amino acids 31 to 293 of SEQ ID NO: 47.

In another embodiment, said fragment comprises or consists of SEQ ID NO: 48.

In another embodiment, said fragment comprises or consists of amino acids 31 to 238 of SEQ ID NO: 48.

In one embodiment, the RBD ligand is isolated from the glycoprotein of feline endogenous virus, and is herein referred as RD114.RBD. In one embodiment, the receptor-binding domain ligand comprises a part or the totality of RD114.RBD and binds to the ASCT1 and/or ASCT2 nutrient transporter(s).

In one embodiment, said RD114.RBD comprises or consists of the amino acid sequence SEQ ID NO: 28 or fragments thereof.

In one embodiment, said fragment comprises or consists of amino acids 19 to 239 of SEQ ID NO: 28.

In one embodiment, said fragment comprises or consists of amino acids 1 to 223, 224, 225, 226, 227, 228, 229, 230, 231, 232, 233, 234, 235, 236, 237 or 238 of SEQ ID NO: 28.

In another embodiment, said fragment comprises or consists of amino acids 19 to 223, 224, 225, 226, 227, 228, 229, 230, 231, 232, 233, 234, 235, 236, 237 or 238 of SEQ ID NO: 28.

In another embodiment, said fragment comprises or consists of SEQ ID NO: 29.

In another embodiment, said fragment comprises or consists of amino acids 19 to 222 of SEQ ID NO: 29.

In one embodiment, said fragment comprises or consists of SEQ ID NO: 49, encoded by the DNA sequence SEQ ID NO: 30.

In another embodiment, said fragment comprises or consists of amino acids 19 to 223 of SEQ ID NO: 49.

In one embodiment, said RD114.RBD comprises or consists of the amino acid sequence SEQ ID NO: 35 or fragments thereof.

In one embodiment, said fragment comprises or consists of amino acids 22 to 331 of SEQ ID NO: 35.

In another embodiment, said fragment comprises or consists of amino acids 1 to 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330 of SEQ ID NO: 35.

In another embodiment, said fragment comprises or consists of amino acids 22 to 281, 282, 283, 284, 285, 286, 287, 288, 289, 290, 291, 292, 293, 294, 295, 296, 297, 298, 299, 300, 301, 302, 303, 304, 305, 306, 307, 308, 309, 310, 311, 312, 313, 314, 315, 316, 317, 318, 319, 320, 321, 322, 323, 324, 325, 326, 327, 328, 329, 330 of SEQ ID NO: 35.

In another embodiment, said fragment comprises or consists of SEQ ID NO: 35, encoded by the DNA sequence SEQ ID NO: 36 or a fragment thereof. Preferably, said fragment of SEQ ID NO: 36 is SEQ ID NO: 50.

In another embodiment, said fragment comprises or consists of amino acids 19 to 222 of SEQ ID NO: 35.

In one embodiment, said RD114.RBD comprises or consists of the amino acid sequence SEQ ID NO: 37 or fragments thereof.

In one embodiment, said fragment comprises or consists of amino acids 1 to 222 of SEQ ID NO: 37.

In one embodiment, said fragment comprises or consists of amino acids 1 to 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221 of SEQ ID NO: 37.

In one embodiment, said fragment comprises or consists of amino acids 19 to 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201, 202, 203, 204, 205, 206, 207, 208, 209, 210, 211, 212, 213, 214, 215, 216, 217, 218, 219, 220, 221 of SEQ ID NO: 37.

In one embodiment, the RBD ligand is isolated from the glycoprotein of baboon endogenous virus, and is herein referred as BaEV.RBD. In one embodiment, the receptor-binding domain ligand comprises a part or the totality of BaEV.RBD and binds to the ASCT1 and/or ASCT2 nutrient transporter(s).

In one embodiment, said BaEV.RBD comprises or consists of the amino acid sequence SEQ ID NO: 39 or fragments thereof.

In one embodiment, said fragment comprises or consists of amino acids 19 to 563 of SEQ ID NO: 39.

In one embodiment, said fragment comprises or consists of amino acids 1 to 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562 of SEQ ID NO: 39.

In one embodiment, said fragment comprises or consists of amino acids 19 to 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562 of SEQ ID NO: 39.

In one embodiment, the RBD ligand is isolated from the glycoprotein of spleen necrosis virus, and is herein referred as SNV.RBD. In one embodiment, the receptor-binding domain ligand comprises a part or the totality of SNV.RBD and binds to the ASCT1 and/or ASCT2 nutrient transporter(s).

In one embodiment, said SNV.RBD comprises or consists of the amino acid sequence SEQ ID NO: 42 or fragments thereof.

In one embodiment, said fragment comprises or consists of amino acids 1 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, 252, 253, 254, 255, 256, 257 of SEQ ID NO: 42.

In another embodiment, said SNV.RBD comprises or consists of the amino acid sequence SEQ ID NO: 43 or fragments thereof.

In another embodiment, said fragment comprises or consists of amino acids 37 to 567 of SEQ ID NO: 43.

In another embodiment, said fragment comprises or consists of amino acids 1 to 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566 of SEQ ID NO: 43.

In another embodiment, said fragment comprises or consists of amino acids 37 to 513, 514, 515, 516, 517, 518, 519, 520, 521, 522, 523, 524, 525, 526, 527, 528, 529, 530, 531, 532, 533, 534, 535, 536, 537, 538, 539, 540, 541, 542, 543, 544, 545, 546, 547, 548, 549, 550, 551, 552, 553, 554, 555, 556, 557, 558, 559, 560, 561, 562, 563, 564, 565, 566 of SEQ ID NO: 43.

In one embodiment, the RBD ligand is isolated from the glycoprotein of simian retrovirus, and is herein referred as SRV.RBD. In one embodiment, the receptor-binding domain ligand comprises a part or the totality of SRV.RBD and binds to the ASCT1 and/or ASCT2 nutrient transporter(s).

In one embodiment, said SRV.RBD comprises or consists of the amino acid sequence SEQ ID NO: 40 or fragments thereof.

In another embodiment, said fragment comprises or consists of amino acids 23 to 251 of SEQ ID NO: 40.

In one embodiment, said fragment comprises or consists of amino acids 1 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250 of SEQ ID NO: 40.

In another embodiment, said fragment comprises or consists of amino acids 23 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250 of SEQ ID NO: 40.

In another embodiment, said fragment is encoded by the DNA sequence SEQ ID NO: 41 or a fragment thereof.

In one embodiment, the RBD ligand is isolated from the glycoprotein of Mason-Pfizer monkey virus, and is herein referred as MPMV.RBD. In one embodiment, the receptor-binding domain ligand comprises a part or the totality of MPMV.RBD and binds to the ASCT1 and/or ASCT2 nutrient transporter(s).

In one embodiment, said MPMV.RBD comprises or consists of the amino acid sequence SEQ ID NO: 44 or fragments thereof.

In another embodiment, said fragment comprises or consists of amino acids 23 to 250 of SEQ ID NO: 44.

In one embodiment, said fragment comprises or consists of amino acids 1 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 of SEQ ID NO: 44.

In another embodiment, said fragment comprises or consists of amino acids 23 to 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249 of SEQ ID NO: 44.

In another embodiment, said fragment is encoded by the DNA sequence SEQ ID NO: 45 or a fragment thereof.

As used herein, "amino acids" are represented by their full name, their three letter code or their one letter code as well known in the art. Amino acid residues in peptides are abbreviated as follows: Phenylalanine is Phe or F; Leucine is Leu or L; Isoleucine is Ile or I; Methionine is Met or M; Valine is Val or V; Serine is Ser or S; Proline is Pro or P; Threonine is Thr or T; Alanine is Ala or A; Tyrosine is Tyr or Y; Histidine is His or H; Glutamine is Gln or Q; Asparagine is Asn or N; Lysine is Lys or K; Aspartic Acid is Asp or D; Glutamic Acid is Glu or E; Cysteine is Cys or C; Tryptophan is Trp or W; Arginine is Arg or R; and Glycine is Gly or G.

As used herein, the term "amino acids" includes both natural and synthetic amino acids, and both D and L amino acids. "Standard amino acid" or "naturally occurring amino acid" means any of the twenty standard L-amino acids commonly found in naturally occurring peptides. "Nonstandard amino acid residue" means any amino acid, other than the standard amino acids, regardless of whether it is prepared synthetically or derived from a natural source. For example, naphtlylalanine can be substituted for tryptophan to facilitate synthesis. Other synthetic amino acids that can be substituted include, but are not limited to, L-hydroxypropyl, L-3,4-dihydroxyphenylalanyl, alpha-amino acids such as L-alpha-hydroxylysyl and D-alpha-methylalanyl, L-alpha-methylalanyl, beta-amino acids, and isoquinolyl.

As used herein, "amino acid" also encompasses chemically modified amino acids, including but not limited to salts, amino acid derivatives (such as amides), and substitutions. Amino acids contained within the polypeptides of the present invention, and particularly at the carboxy- or amino-terminus, can be modified by methylation, amidation, acetylation or substitution with other chemical groups which can change the polypeptide's circulating half-life without adversely affecting their activity. Additionally, a disulfide linkage may be present or absent in the polypeptides of the invention.

The RBD ligands of the invention may comprise naturally standard amino acids or nonstandard amino acids. Polypeptide mimetics include polypeptides having the following modifications: i) polypeptides wherein one or more of the peptidyl -C(O)NR- linkages (bonds) have been replaced by a non-peptidyl linkage such as a -CH₂-carbamate linkage (-CH₂OC(O)NR-), a phosphonate linkage, a -CH₂-sulfonamide (-CH₂-S(O)₂NR-) linkage, a urea (-NHC(O)NH-) linkage, a -CH₂-secondary amine linkage, or with an alkylated peptidyl linkage (-C(O)NR-) wherein R is C₁-C₄ alkyl; ii) polypeptides wherein the N-terminus is derivatized to a -NRR¹ group, to a -NRC(O)R group, to a -NRC(O)OR group, to a -NRS(O)₂R group, to a -NHC(O)NHR group where Rand R¹ are hydrogen or C₁-C₄ alkyl with the proviso that R and R¹ are not both hydrogen; iii) polypeptides wherein the C terminus is derivatized to -C(O)R² where R² is selected from the group consisting of C₁-C₄ alkoxy, and -NR³R⁴ where R³ and R⁴ are independently selected from the group consisting of hydrogen and C₁-C₄ alkyl.

According to a preferred embodiment, receptor-binding domain ligands are selected from the group comprising the sequences SEQ ID NO: 1, 2, 4, 5, 28, 29, 35, 37, 38, 39, 40, 42, 43, and 44, fragments and variants thereof, more preferably selected from the group comprising the sequences SEQ ID NO: 2, 5, 29, 35, 37, 38, 39, 40, 42, 43, and 44, fragments and variants thereof. According to another embodiment, receptor-binding domain ligands are encoded by a DNA sequence selected from the group comprising the sequences SEQ ID NO: 3, 6, 30, 36, 41, and 45, variants and fragments thereof.

In one embodiment, the RBD ligand comprises or consists of a sequence presenting a sequence identity of at least 70% with one of the sequences SEQ ID NO: 1, 2, 4, 5, 28, 29, 35, 37, 38, 39, 40, 42, 43, and 44, preferably a sequence identity of at least about 75, 80, 85, 90, 91, 92, 93, 94, 95, 96, 97, 98, 99% or more with one of the sequences SEQ ID NO: 1, 2, 4, 5, 28, 29, 35, 37, 38, 39, 40, 42, 43, and 44.

In another embodiment, the RBD ligand is encoded by a DNA sequence presenting a sequence identity of at least 70% with one of the sequences SEQ ID NO: 3, 6, 30, 36, 41, and 45 preferably a sequence identity of at least about 75, 80, 85, 90, 91, 92, 93, 94, 95, 96, 97, 98, 99% or more with one of the sequences SEQ ID NO: 3, 6, 30, 36, 41, and 45.

In one embodiment, the RBD ligand is a variant of one of the polypeptides having the sequences SEQ ID NO: 1, 2, 4, 5, 28, 29, 35, 37, 38, 39, 40, 42, 43, and 44.

A polypeptide "variant" as the term is used herein, is a polypeptide that typically differs from a polypeptide specifically disclosed herein in one or more substitutions, deletions, additions and/or insertions. Such variants may be naturally occurring or may be synthetically generated, for example, by modifying one or more of the above polypeptide sequences and evaluating one or more biological activities of the polypeptide as described herein and/or using any of a number of techniques well known in the art. Modifications may be made in the structure of polypeptides and still obtain a functional molecule that encodes a variant or derivative polypeptide with desirable characteristics.

When it is desired to alter the amino acid sequence of a polypeptide to create an equivalent, or even an improved, variant or portion of a ligand of the invention, one skilled in the art will typically change one or more of the codons of the encoding DNA sequence. For example, certain amino acids may be substituted by other amino acids in a protein structure without appreciable loss of its ability to bind cell surface receptor, preferably cell surface nutrient transporters. Since it is the binding capacity and nature of a protein that defines that protein's biological functional activity, certain amino acid sequence substitutions can be made in a protein sequence, and, of course, its underlying DNA coding sequence, and nevertheless obtain a protein with similar properties. It is thus contemplated that various changes may be made in the peptide sequences, or corresponding DNA sequences that encode said peptides without appreciable loss of their biological utility or activity. In many instances, a polypeptide variant will contain one or more conservative substitutions. A "conservative substitution" is one in which an amino acid is substituted by another amino acid that has similar properties, such that one skilled in the art of peptide chemistry would expect the secondary structure and hydropathic nature of the polypeptide to be substantially unchanged. As outlined above, amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine. Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include histidine, lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his.

As used herein, the term "conservative amino acid substitution" may further be defined as an amino acid exchange within one of the following five groups:
I. Small aliphatic, nonpolar or slightly polar residues: Ala, Ser, Thr, Pro, Gly;
II. Polar, negatively charged residues and their amides: Asp, Asn, Glu, Gln;
III. Polar, positively charged residues: His, Arg, Lys;
IV. Large, aliphatic, nonpolar residues: Met, Leu, Ile, Val, Cys;
V. Large, aromatic residues: Phe, Tyr, Trp.

A variant may also, or alternatively, contain nonconservative changes. In a preferred embodiment, variant polypeptides differ from a native sequence by substitution, deletion or addition of 1, 2, 3, 4, 5, 6, 7, 8, 9 or 10 amino acids. Variants may also (or alternatively) be modified by, for example, the deletion or addition of amino acids that have minimal influence on the immunogenicity, secondary structure and hydropathic nature of the polypeptide.

In one embodiment, a variant of SEQ ID NO: 1, 2, 4, 5, 28, 29, 35, 37, 38, 39, 40, 42, 43, and 44 is capable of binding to a cell surface receptor, preferably to a cell surface nutrient transporter with an affinity at least equivalent to the one of SEQ ID NO: 1, 2, 4, 5, 28, 29, 35, 37, 38, 39, 40, 42, 43, and 44 respectively.

In one embodiment, a variant of SEQ ID NO: 1, 2, 4, 5, 28, 29, 35, 37, 38, 39, 40, 42, 43, and 44 comprises conservative amino acid substitutions as compared to the sequence of SEQ ID NO: 1, 2, 4, 5, 28, 29, 35, 37, 38, 39, 40, 42, 43, and 44 respectively, such as, for example, 1, 2, 3, 4, 5, 6, 7, 8, 9 or 10 conservative amino acid substitutions.

In another embodiment, a variant of SEQ ID NO: 1, 2, 4, 5, 28, 29, 35, 37, 38, 39, 40, 42, 43, and 44 is a polypeptide wherein 1, 2, 3, 4, 5, 6, 7, 8, 9 or 10 amino acids from the sequence of SEQ ID NO: 1, 2, 4 5, 28, 29, 35, 37, 38, 39, 40, 42, 43, and 44 respectively is/are absent, or substituted by any amino acid, or wherein 1, 2, 3, 4, 5, 6, 7, 8, 9 or 10 amino acids (either contiguous or not) is/are added.

In one embodiment of the invention, the RBD ligands as described here above are modified by means well-known in the art, for instance by the addition of one or more functional group such as a phosphate, acetate, lipid or carbohydrate group, and/or by the addition of one or more protecting group.

For example, the RBD ligands can be modified by the addition of one or more functional groups such as phosphate, acetate, or various lipids and carbohydrates. The RBD ligands of the invention can also exist as polypeptide derivatives. The term "polypeptide derivative" refers to compound having an amino group (--NH--), and more particularly, a peptide bond. Polypeptides may be regarded as substituted amides. Like the amide group, the peptide bond shows a high degree of resonance stabilization. The C--N single bond in the peptide linkage has typically about 40 percent double-bond character and the C=O double bond about 40 percent single-bond character. "Protecting groups" are those groups that prevent undesirable reactions (such as proteolysis) involving unprotected functional groups. Specific examples of amino protecting groups include formyl; trifluoroacetyl; benzyloxycarbonyl; substituted benzyloxycarbonyl such as (ortho- or para-) chlorobenzyloxycarbonyl and (ortho- or para-) bromobenzyloxycarbonyl; and aliphatic oxycarbonyl such as t-butoxycarbonyl and t-amiloxycarbonyl. The carboxyl groups of amino acids can be protected through conversion into ester groups. The ester groups include benzyl esters, substituted benzyl esters such as methoxybenzyl ester; alkyl esters such as cyclohexyl ester, cycloheptyl ester or t-butyl ester. The guanidino moiety may be protected by nitro; or arylsulfonyl such as tosyl, methoxybenzensulfonyl or mesitylenesulfonyl, even though it does not need a protecting group. The protecting groups of imidazole include tosyl, benzyl and dinitrophenyl. The indole group of tryptophan may be protected by formyl or may not be protected.

The modification of the RBD ligands aims in particular to improve their life time *in vivo.* One type of modification is the addition to the N or C termini of the RBD ligands of polyethylene glycol (PEG). PEG is known by the person skilled in the art to have many properties that make it an ideal carrier for polypeptides such as high water solubility, high mobility in solution and low immunogenicity. This modification also protects the polypeptides from exopeptidases and therefore increases their overall stability *in vivo.*

The other modifications used to prevent degradation of the polypeptides by endopeptidases or exopeptidases include N-terminal modifications such as acetylation or glycosylation, C-terminal modifications such as amidation and use of unnatural amino acids (β-amino and α-trifluoromethyl amino acids) at particular sites within the polypeptides.

Another alternative to increase polypeptide molecular size is the genetic fusion of the polypeptides to the Fc domain of human immunoglobulin (including, for example, IgA, IgM and IgG) or the fusion of the polypeptides to albumin.

In one embodiment, the RBD ligand is a fusion protein comprising a part or the totality of a RBD fused to a detection tag, such as, for example, a Fc fragment or a GFP. Examples of Fc fragments include, but are not limited to, rabbit Fc fragment (amino acid sequence SEQ ID NO: 9, encoded by SEQ ID NO: 10), mouse Fc fragment (amino acid sequence SEQ ID NO: 11, encoded by SEQ ID NO: 12) and human Fc fragment such as huIgG2 Fc (encoded by SEQ ID NO: 27).

In one embodiment, said fusion protein comprises a spacer between a part or the totality of a RBD and the detection tag. In one embodiment, said spacer comprises or consists of amino acid sequence GS. In one embodiment, said spacer is encoded by the DNA sequence GGATCC, corresponding to a BamHI restriction site.

In one embodiment, the receptor-binding domain ligand is selected from the group comprising HERV-W.RBD fused to a mouse Fc fragment (such as, for example SEQ ID NO: 51, encoded by the DNA sequence SEQ ID NO: 7), RD114.RBD fused to a mouse Fc fragment (such as, for example SEQ ID NO: 54; or SEQ ID NO: 55, encoded by the DNA sequence SEQ ID NO: 31) and Xeno.RBD fused to a rabbit Fc fragment (such as, for example SEQ ID NO: 52; or SEQ ID NO: 53, encoded by the DNA sequence SEQ ID NO: 8).

In one embodiment, the receptor-binding ligand of the invention is coupled with at least one contrast agent. Non-limiting examples of contrast agents are listed hereinabove. In one embodiment, the receptor-binding ligand of the invention is coupled with I-125.

The RBD ligands described herein can be produced synthetically by chemical synthesis or enzymatic synthesis as it is well known in the art. Alternatively, nucleotide sequences encoding the polypeptides of the invention can be introduced into a protein expression vector and produced in a suitable host organism (e.g., bacteria, insect cells, etc.), then purified. In one embodiment, the receptor-binding domain ligand is obtained by a cloning method, such as, for example, using any production system known in the art, such as, for example, *E. coli,* yeast, baculovirus-insect cell, or mammalian cells such as HEK or CHO expression system.

An additional polypeptide ("tag") can be added on for the purpose of purifying or identifying or purifying the polypeptides. Protein tags make it possible, for example, for the polypeptides to be adsorbed, with high affinity, to a matrix, and for the matrix then to be washed stringently with suitable buffers without the complex being eluted to any significant extent, and for the adsorbed complex subsequently to be eluted selectively. Examples of protein tags which are known to the skilled person are a (His)₆ tag, a Myc tag, a FLAG tag, a hemagglutinin tag, a glutathione transferase (GST) tag, intein having an affinity chitin- binding tag or maltose-binding protein (MBP) tag. These protein tags can be located N- terminally, C -terminally and/or internally.

In one embodiment, the sequence of the RBD ligand is fused in N-terminal to a peptide signal sequence allowing the secretion of said RBD ligand. Examples of peptide signal sequences include, but are not limited to, human IL-2 peptide signal (SEQ ID NO: 13), human albumin peptide signal (SEQ ID NO: 14), human chymotrypsinogen peptide signal (SEQ ID NO: 15), human trypsinogen-2 peptide signal (SEQ ID NO: 16), Gaussia luciferase peptide signal (SEQ ID NO: 17), and mouse IgM peptide signal (SEQ ID NO: 18).

In one embodiment, the method of the invention comprises measuring the binding of HERV-W.RBD to ASCT1, ASCT2 or ASCT1 and ASCT2.

In one embodiment, the method of the invention comprises measuring the binding of RD114.RBD to ASCT1, ASCT2 or ASCT1 and ASCT2.

In one embodiment, the method of the invention comprises measuring the binding of BaEV.RBD to ASCT1, ASCT2 or ASCT1 and ASCT2.

In one embodiment, the method of the invention comprises measuring the binding of SNV.RBD to ASCT1, ASCT2 or ASCT1 and ASCT2.

In one embodiment, the method of the invention comprises measuring the binding of SRV.RBD to ASCT1, ASCT2 or ASCT1 and ASCT2.

In one embodiment, the method of the invention comprises measuring the binding of MPMV.RBD to ASCT1, ASCT2 or ASCT1 and ASCT2.

In another embodiment, the method of the invention comprises measuring the binding of Xeno.RBD to XPR1.

In another embodiment, the method of the invention does not consist in measuring the binding of RBD derived from human T-lymphotropic virus (HTLV)1, HTLV2 or HTLV4 or from simian T-lymphotropic virus (STLV)1, STLV2 or STLV3 to GLUT1.

The present invention further relates to a method for detecting a pancreatic cancer cell, wherein said method comprises determining or measuring the binding of at least one RBD ligand on said cell, wherein said RBD ligand is selected from the group comprising HERV-W.RBD, RD114.RBD, BaEV.RBD, SNV.RBD, SRV.RBD, MPMV.RBD, Xeno.RBD, variants and fragments thereof.

In one embodiment, the method of the invention comprises comparing the binding of the at least one RBD ligand with a reference value.

Another object of the invention is a RBD ligand coupled with at least one contrast agent. Preferably, said RBD ligand is HERV-W RBD, RD114.RBD, BaEV.RBD, SNV.RBD, SRV.RBD, MPMV.RBD, Xeno.RBD, variants or fragments thereof, more preferably HERV-W.RBD, RD114.RBD or Xeno.RBD, variants and fragments thereof (as described hereinabove) coupled with at least one contrast agent (as described hereinabove). In one embodiment, the at least one contrast agent is a radiolabeled agent. In one embodiment, the at least one contrast agent is I-125.

In one embodiment, the at least one RBD ligand coupled with at least one contrast agent may be used as a probe for medical imaging. In one embodiment, the at least one RBD ligand coupled with a radiolabeled agent may be used as a probe for medical imaging. In one embodiment, the at least one RBD ligand coupled with I-125 may be used as a probe for medical imaging.

Methods for coupling at least one contrast agent to a RBD ligand are well known in the state of the art. For instance, the at least one contrast agent may be bound covalently or non-covalently.

For example, technics to couple polypeptides to I-125 are well known in the state of the art. An example of such a method is the following: iodine present in a reduced form (NaI) reacts with the phenol group of a tyrosine or with the side chain of a histidine residue. These groups are pre-oxidized with an oxidizing agent (iodogen). The peptides preparation (100 µg for 1 mci = 37 MBq) is then added to an iodogen solution and incubated for 10 minutes at 4°C. The reaction is stopped using a stop solution comprising for example 200 µL of PBS with sodium azide per marking. In parallel, a mouse serum is added onto a PD10 column. Then the reaction solution is added onto the PD10 column and the peptide coupled with the iodine is collected.

In one embodiment of the invention, the RBD ligand coupled with at least one contrast agent of the invention is for use as a tracer. The term "tracer", as used herein, refers to a recognition agent providing insight into cancer location, cancer progression and structure for pre-, intra- and post-operative surgery.

In one embodiment, said method is for use in pre-, intra-, or post-operative surgery. In another embodiment, said method is for use in fluorescence guided surgery.

In one embodiment, the detection of said at the least one RBD ligand binding to the pancreatic cancer cells is carried out 4h, 6h, 12h, 18h, 24h, 36h, 48h, or 96h after administration of the at least one RBD ligand coupled with at least one contrast agent to the subject. The skilled artisan would determine the correct read out depending on the contrast agent used.

Examples of specific medical imaging technics methods that may be used are well known to the skilled artisan and include for instance computer assisted tomography (CAT), magnetic resonance spectroscopy (MRS), magnetic resonance imaging (MRI), positron emission tomography (PET) or single-photon emission computed tomography (SPECT) and are described in Boonstra *et al.* 2014.

In one embodiment, the RBD ligand is coupled with a radiolabeled agent or a fluorescent agent as described herein. Preferably, the radiolabeled agent or the fluorescent agent for targeting includes but is not limited to: I-125, I-131, F-18 (i.e. 18-F-fluoro-2-deoxy-D-glucose, 18-F-fluoro-17-estradiol), 11-C-acetate, 99 mTc, 0-15, N-13, Br-76, In-111, Cu-64, Ga-68, Zr-89, ZW800-1, Cy5.5, IRDye800CW.

As used herein, the term "reference" broadly encompasses any suitable reference expression level which may be used as a basis for comparison with respect to the measured expression level. In one embodiment, the reference is constructed using algorithms and/or other methods of statistical and hierarchical classification. In another aspect, the reference expression level is stored in a database to provide a stored expression level and the stored expression level is used to determine the difference in the expression level. The database may, for example, be stored on a computer or a server.

In one embodiment, the reference expression level is an index value or is derived from one or more risk prediction algorithms or computed indices for the presence of pancreatic cancer cells. A reference expression level can be relative to a number or value derived from population studies, including without limitation, such populations of subjects having similar age range, subjects in the same or similar ethnic group.

In one embodiment of the invention, the reference expression level is the expression level measured in a population of patients diagnosed with a pancreatic cancer. In one embodiment, an equivalence (i.e. an absence of difference) between the measured expression level and the reference expression level, or a measured expression level superior to the reference expression level may be indicative of the presence of pancreatic cancer cells.

In one embodiment of the invention, the reference expression level is the expression level measured in a population of substantially healthy subjects, i.e. in a population of subjects not diagnosed with a pancreatic cancer. In one embodiment, a measured expression level superior to the reference expression level may be indicative of the presence of pancreatic cancer cells.

In the present invention, two numeric values, in particular two expression levels, are considered as different if the first numeric value is higher (such as, for example, the first numeric value is about 20% higher than the second one, preferably is about 30, 40, 50, 60, 70, 80, 90% or more higher than the second one) or lower than the second one (such as, for example, the second numeric value is about 20% lower than the second one, preferably is about 30, 40, 50, 60, 70, 80, 90% or more lower than the second one).

In one embodiment, the reference value is a personalized reference, determined earlier in the same subj ect (such as, for example, before receiving a treatment for treating pancreatic cancer).

The term "pancreatic cancer" as used herein refers to abnormal proliferation of pancreatic cells. In particular, pancreatic cancer cells express, preferably overexpress, the nutrient cell transporters ASCT1 and/or ASCT2 and/or XPR1.

In one embodiment, pancreatic cancer cells do not overexpress GLUT1.

Pancreatic cancer can be divided into two general groups. The vast majority of cases (about 99%) occur in the part of the pancreas which produces digestive enzymes, known as the exocrine group. The small minority of these cancers arises in the hormone-producing tissue of the pancreas and is thus known as the endocrine group.

Examples of pancreatic cancers of the exocrine group include, but are not limited to, adenocarcinoma, acinar cell carcinoma, cystadenocarcinoma, pancreatoblastoma, adenosquamous carcinoma, signet ring cell carcinoma, hepatoid carcinoma, colloid carcinoma, undifferentiated carcinoma, and undifferentiated carcinoma with osteoclastlike giant cells, solid pseudopapillary tumor, and pancreatic mucinous cystic neoplasm.

Examples of pancreatic cancers of the neuroendocrine group include, but are not limited to, pancreatic neuroendocrine tumor, malign pancreatic neuroendocrine tumor, benign pancreatic neuroendocrine tumor, insulinoma and gastrinoma.

The present disclosure relates to a method for the diagnosis of a pancreatic cancer, which is not covered by the claimed invention, comprising the steps of:
a. contacting an effective amount of at least one RBD ligand selected from HERV-W.RBD, RD114.RBD, BaEV.RBD, SNV.RBD, SRV.RBD, MPMV.RBD, Xeno.RBD, variants and fragments thereof (preferably HERV-W.RBD, RD114.RBD, and Xeno.RBD, variants and fragments thereof), to a tissue, an organ or a cell;
b. detecting and/or quantifying the binding of the at least one RBD ligand to at least one cell surface receptor, preferably at least one cell surface nutrient transporter on said tissue or organ or cell.

In one embodiment not covered by the claimed invention, the method for the diagnosis of a pancreatic cancer comprises the steps of:
a. contacting an effective amount of at least one RBD ligand selected from HERV-W.RBD, RD114.RBD, BaEV.RBD, SNV.RBD, SRV.RBD, MPMV.RBD and Xeno.RBD, to a tissue, an organ or a cell;
b. detecting and/or quantifying the binding of the at least one RBD ligand to at least one cell surface receptor selected from the group consisting of ASCT1, ASCT2 and XPR1 on said tissue or organ or cell.

In one embodiment, the at least one RBD ligand is a RBD ligand fused to at least one contrast agent as described hereinabove. In one embodiment, the at least one RBD ligand is a RBD ligand fused to at least one radiolabeled agent. In one embodiment, the at least one radiolabeled agent is 1-125.

In one embodiment, the ligand of the invention is for monitoring a pancreatic cancer in a subject. The term "monitoring" as used herein refers to the determination of the amount of pancreatic cancer cells in the body of a subject as a function of time, such as, for example, before, during and after an anti-cancer therapy.

The term "anti-cancer therapy" as used herein refers to chemotherapy, radiation, surgery, immunotherapy, and drugs known to the skilled artisan as anti-cancer drugs.

In one embodiment not covered by the claimed invention, the method of monitoring comprises comparing two expression levels, such as, for example, an expression level (of ASCT1 and/or ASCT2 and/or XPR1) measured before treatment with an expression level (of ASCT1 and/or ASCT2 and/or XPR1) measured after treatment.

In one embodiment, a decreased expression level of ASCT1 and/or ASCT2 and/or XPR1 after treatment is indicative of the efficacy of the treatment.

In one embodiment, an expression level of ASCT1 and/or ASCT2 and/or XPR1 after treatment equivalent or superior to the one measured before treatment is indicative of the absence of efficacy of the treatment.

In one embodiment not covered by the claimed invention, the absence of detection of the at least one receptor, preferably of the at least one cell surface nutrient cell transporter, preferably of the at least one cell surface nutrient cell transporter selected from the group consisting of ASCT1, ASCT2 and XPR1 on a tissue, an organ or a cell after an anti-cancer therapy, is indicative of a remission.

The present disclosure further relates to a composition not covered by the claimed invention comprising at least one RBD ligand coupled with at least one contrast agent, preferably coupled with at least one radiolabeled agent, preferably coupled with I-125, as described hereinabove.

The present disclosure further relates to a pharmaceutical composition not covered by the claimed invention comprising, consisting or consisting essentially of at least one RBD ligand coupled with at least one contrast agent, preferably coupled with at least one radiolabeled agent, preferably coupled with I-125, as described hereinabove and at least one pharmaceutically acceptable excipient.

The present disclosure further relates to a medicament not covered by the claimed invention comprising, consisting or consisting essentially of at least one RBD ligand coupled with at least one contrast agent, preferably coupled with at least one radiolabeled agent, preferably coupled with I-125, as described hereinabove.

As used herein, the term "consisting essentially of', with reference to a pharmaceutical composition or medicament, means that the at least one RBD ligand of the invention is the only one therapeutic agent or agent with a biologic activity within said pharmaceutical composition or medicament.

The present disclosure also relates to a diagnostic composition not covered by the claimed invention comprising, consisting or consisting essentially of at least one RBD ligand coupled with at least one contrast agent, preferably coupled with at least one radiolabeled agent, preferably coupled with I-125, as described hereinabove and at least one pharmaceutically acceptable excipient.

In one embodiment not covered by the claimed invention, the diagnostic composition is for diagnosing pancreatic cancer or for monitoring pancreatic cancer, according to the methods of the invention as described hereinabove.

Pharmaceutically acceptable excipients include water, saline, Ringer's solution, dextrose solution, and solutions of ethanol, glucose, sucrose, dextran, mannose, mannitol, sorbitol, polyethylene glycol (PEG), phosphate, acetate, gelatin, collagen, Carbopol^{®}, vegetable oils, and the like. One may additionally include suitable preservatives, stabilizers, antioxidants, antimicrobials, and buffering agents, such as, for example, BHA, BHT, citric acid, ascorbic acid, tetracycline, and the like.

Other examples of pharmaceutically acceptable excipients that may be used in the composition of the invention include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene- polyoxypropylene- block polymers, polyethylene glycol and wool fat.

In addition, pharmaceutically acceptable excipients may comprise some excipients, such as, for example, surfactants (e.g. hydroxypropylcellulose); suitable carriers, such as, for example, solvents and dispersion media containing, for example, water, ethanol, polyol (e.g. glycerol, propylene glycol, and liquid polyethylene glycol, and the like), suitable mixtures thereof, and vegetable oils, such as, for example, peanut oil and sesame oil; isotonic agents, such as, for example, sugars or sodium chloride; coating agents, such as, for example, lecithin; agents delaying absorption, such as, for example, aluminum monostearate and gelatin; preservatives, such as, for example, benzalkonium chloride, benzethonium chloride, chlorobutanol, thimerosal and the like; buffers, such as, for example, boric acid, sodium and potassium bicarbonate, sodium and potassium borates, sodium and potassium carbonate, sodium acetate, sodium biphosphate and the like; tonicity agents, such as, for example, dextrose, potassium chloride, propylene glycol, sodium chloride; antioxidants and stabilizers, such as, for example, sodium bisulfite, sodium metabisulfite, sodium thiosulfite, thiourea and the like; nonionic wetting or clarifying agents, such as, for example, polysorbate 80, polysorbate 20, poloxamer 282 and tyloxapol; viscosity modifying agents, such as, for example dextran 40, dextran 70, gelatin, glycerin, hydroxyethylcellulose, hydroxmethylpropylcellulose, lanolin, methylcellulose, petrolatum, polyethylene glycol, polyvinyl alcohol, polyvinylpyrrolidone, carboxymethylcellulose; and the like.

The present disclosure also relates to a pharmaceutical composition not covered by the claimed invention for treating or for use in treating pancreatic cancer.

The present disclosure also relates to a medicament not covered by the claimed invention for treating or for use in treating pancreatic cancer.

In one embodiment, said RBD ligand coupled with at least one contrast agent, preferably coupled with at least one radiolabeled agent, preferably coupled with I-125, is encapsulated. The encapsulation of the RBD ligand coupled with at least one contrast agent may avoid any degradation. The technics of encapsulation are well known in the state of the art.

Examples of capsule include but are not limited to: phospholipids, polymers, liposomes and quantum dots.

In one embodiment, the RBD ligand is encapsulated with a therapeutic agent to be specifically administered to pancreatic cancer cells within the subject's body.

In one embodiment, the RBD ligand, the RBD ligand coupled with at least one contrast agent or the diagnostic composition of the invention or the pharmaceutical composition of the invention or the medicament of the invention is to be administered at a dose determined by the skilled artisan and personally adapted to each subject.

In one embodiment of the invention, the RBD ligand, the RBD ligand coupled with at least one contrast agent or the diagnostic composition of the invention or the pharmaceutical composition of the invention or the medicament of the invention is to be administered at an effective amount.

It will be understood that the usage of the RBD ligand, the RBD ligand coupled with at least one contrast agent or the diagnostic composition of the invention will be decided by the attending physician within the scope of sound medical judgment. The specific effective amount for any particular patient will depend upon a variety of factors including the specific composition employed, the age, body weight, general health, sex and diet of the subject; the time of administration, route of administration, and like factors well known in the medical arts.

In one embodiment, the RBD ligand, the RBD ligand coupled with at least one contrast agent or the diagnostic composition, pharmaceutical composition or medicament of the invention is to be administered by injection, orally, topically, nasally, buccally, rectally, vaginally, intratracheally, by endoscopy, transmucosally, or by percutaneous administration.

In one embodiment, the RBD ligand, the RBD ligand coupled with at least one contrast agent or the diagnostic composition, pharmaceutical composition or medicament of the invention is to be administered by injection, preferably systemically injected. Examples of formulations adapted to systemic injections include, but are not limited to: liquid solutions or suspensions, solid forms suitable for solution in, or suspension in, liquid prior to injection. Examples of systemic injections include, but are not limited to, intravenous, subcutaneous, intramuscular, intradermal, intravitreal, and intraperitoneal injection, or perfusion. In another embodiment, when injected, the composition, the pharmaceutical composition or the medicament of the invention is sterile. Methods for obtaining a sterile pharmaceutical composition include, but are not limited to, GMP synthesis (GMP stands for "Good manufacturing practice").

In one embodiment, the RBD ligand, the RBD ligand coupled with at least one contrast agent or the diagnostic composition, pharmaceutical composition or medicament of the invention is to be orally administered. Examples of formulations adapted to oral administration include, but are not limited to: solid forms, liquid forms and gels. Examples of solid forms adapted to oral administration include, but are not limited to, pill, tablet, capsule, soft gelatine capsule, hard gelatine capsule, caplet, compressed tablet, cachet, wafer, sugar-coated pill, sugar coated tablet, or dispersing/or disintegrating tablet, powder, solid forms suitable for solution in, or suspension in, liquid prior to oral administration and effervescent tablet. Examples of liquid form adapted to oral administration include, but are not limited to, solutions, suspensions, drinkable solutions, elixirs, sealed phial, potion, drench, syrup and liquor.

In another embodiment, the RBD ligand, the RBD ligand coupled with at least one contrast agent or the diagnostic composition, pharmaceutical composition or medicament of the invention is to be topically administered. Examples of formulations adapted to topical administration include, but are not limited to, sticks, waxes, creams, lotions, ointments, balms, gels, masks, leave-on washes and/or the like.

Depending on the organ targeted, the skilled artisan can determine the technology needed for the introduction of the RBD ligand in the targeted organ.

In one embodiment, the RBD ligand, the RBD ligand coupled with at least one contrast agent or the diagnostic composition, pharmaceutical composition or medicament of the invention is to be administered in a sustained-release form. In another embodiment, the RBD ligand, the RBD ligand coupled with at least one contrast agent or the diagnostic composition of the invention comprises a delivery system that controls the release of the agent.

The "targeted organ" as used herein may refer to a pancreas affected or suspected to be affected by cancer.

In one embodiment, a therapeutically effective amount of pharmaceutical composition or medicament of the invention is administered at least once a day, twice a day, or at least three times a day.

In another embodiment, a therapeutically effective amount of the pharmaceutical composition or medicament of the invention is administered every two, three, four, five, or six days.

In another embodiment, a therapeutically effective amount of the pharmaceutical composition or medicament of the invention is administered every week, twice a week, every two weeks, or once a month.

In another embodiment, a therapeutically effective amount of the RBD ligand, the RBD ligand coupled with at least one contrast agent or the diagnostic composition of the invention or the pharmaceutical composition of the invention or the medicament of the invention is administered every month for a period at least 2; 3; 4; 5; or 6 months.

In another embodiment, a therapeutically effective amount of the RBD ligand, the RBD ligand coupled with at least one contrast agent or the diagnostic composition of the invention or the pharmaceutical composition of the invention or the medicament of the invention ranges from about 1 µg to 5 g.

In another embodiment, a therapeutically effective amount of the RBD ligand, the RBD ligand coupled with at least one contrast agent or the diagnostic composition of the invention or the pharmaceutical composition of the invention or the medicament of the invention is to be administered ranges from about 0.1 µg/kg to 1 g/kg.

In another embodiment, the RBD ligand, the RBD ligand coupled with at least one contrast agent or the diagnostic composition of the invention or the pharmaceutical composition of the invention or the medicament of the invention as described here above is to be administered in combination with another treatment for pancreatic cancer.

Examples of anti-pancreatic cancer agent comprise but are not limited to: chemotherapy, radiation, surgery, protein kinases inhibitors, microtubules inhibitors, anti-metabolite agents a tumor vaccine or an immunostimulatory antibody.

In one embodiment, the subject is affected, preferably is diagnosed with a pancreatic cancer. In another embodiment, the subject of the invention is at risk of developing a pancreatic cancer. Examples of risk factor include, but are not limited to, familial history of pancreatic cancer, genetic factors, smoking, obesity, diabetes and alcohol.

In another embodiment, the subject of the invention is in a remission stage following a pancreatic cancer.

Another object of the present invention is a kit for implementing the method of the invention, wherein said kit comprises means for measuring the expression level of at least one cell surface receptor, preferably of at least one cell surface nutrient transporter, preferably of at least one cell surface nutrient transporter selected from the group consisting of ASCT1, ASCT2 and XPR1.

In one embodiment, the expression level of at least one cell surface nutrient transporter is assessed at the RNA level, and the kit of the invention may comprise means for total RNA extraction, means for reverse transcription of total RNA, and means for quantifying the expression of RNA of at least one cell surface nutrient transporter, preferably ASCT1, ASCT2 and/or XPR1. In one embodiment, the means for quantifying the expression of RNA of at least one cell surface nutrient transporter, preferably ASCT1, ASCT2 and/or XPR1 are PCR or qPCR primers specific for said cell surface nutrient transporter, preferably ASCT1, ASCT2 and/or XPR1. Examples of PCT or qPCR primers are described hereinabove. In one embodiment, the kit also comprises reagents for carrying out a quantitative PCR (such as, for example, buffers, enzyme, and the like). In one embodiment, the kit of the invention may also comprise means for detecting the expression level of at least one normalization gene at the RNA level.

In another embodiment, the expression level of at least one cell surface nutrient transporter is assessed at the protein level, and the kit of the invention may comprise means for detecting the at least one cell surface nutrient transporter selected from ASCT1, ASCT2 and/or XPR1. In one embodiment, said means for detecting the at least one cell surface nutrient transporter is an antibody specific of said at least one cell surface nutrient transporter selected from ASCT1, ASCT2 and/or XPR1. In another embodiment, said means for detecting the at least one cell surface nutrient transporter is a RBD as defined in the present invention and specific of the at least one cell surface nutrient transporter. In one embodiment, the kit of the invention may also comprise means for detecting the expression level of at least one normalization protein or of a protein allowing monitoring protein expression. Examples of protein allowing monitoring protein expression include but are not limited to: MHC class I and CD326 (also known as human epithelial antigen (HEA), epithelial cell adhesion molecule (EpCAM), or epithelial-specific antigen (ESA)).

In one embodiment, the kit of the invention comprises at least one RBD ligand coupled with at least one contrast agent as described here above. In one embodiment, said RBD ligand is selected from HERV-W.RBD, RD114.RBD, BaEV.RBD, SNV.RBD, SRV.RBD, MPMV.RBD, or Xeno.RBD, variants and fragments thereof, preferably from HERV-W.RBD, RD114.RBD, and Xeno.RBD, variants and fragments thereof.

By "kit" is intended any manufacture (e.g., a package or a container) comprising at least one reagent (such as, for example, a RBD ligand coupled with at least one contrast agent) for specifically detecting the expression of the cell nutrient transporter. The kit may be promoted, distributed, or sold as a unit for performing the methods of the present invention. Furthermore, any or all of the kit reagents may be provided within containers that protect them from the external environment, such as in sealed and sterile containers. The kits may also contain a package insert describing the kit and methods for its use.

In one embodiment of the invention, the RBD ligand is coupled with at least one contrast agent, preferably coupled with at least one radiolabeled agent, preferably coupled with I-125, and may be used for *in vivo* diagnosis by medical imaging.

### BRIEF DESCRIPTION OF THE DRAWINGS

**Figure 1** is a set of graphs showing flow cytometry analysis following the incubation of H2.RBD.GFP with MiaPaca2, Panclwt, BxPC3 and CFPAC cell lines.
**Figure 2** is a set of graphs showing flow cytometry analysis following the incubation of Xeno.RBD.RFC with MiaPaca2, Panclwt, BxPC3 and CFPAC cell lines.
**Figure 3** is a set of graphs showing flow cytometry analysis following the incubation of HERV-W.RBD.MFC with MiaPaca2, Panc1wt, BxPC3 and CFPAC cell lines.
**Figure 4** is a graph showing the recognition *in vivo* of different pancreatic tumor cell lines by I-125.HERV-W.RBD.MFC.
**Figure 5** is a graph showing the recognition *in vivo* of different pancreatic tumor cell lines by I-125.Xeno.RBD.RFC.
**Figure 6** is a graph showing the recognition *in vivo* of different pancreatic tumor cell lines by I-125.H2.RBD.GFP.
**Figure 7** is a set of images showing the recognition *in vivo* of a mouse xenografted with human pancreatic tumors or not (control) by I-125.HERV-W.RBD.MFC.
**Figure 8** is a set of images showing the recognition *in vivo* of a mouse xenografted with human pancreatic tumors or not (control) by I-125.Xeno.RBD.RFC.
**Figure 9** is a set of images showing the recognition *in vivo* of a mouse xenografted with human pancreatic tumors or not (control) by I-125.H2.RBD.GFP.

### EXAMPLES

The present invention is further illustrated by the following examples.

### Example 1

### Materials and Methods

MiaPaca2 and Panc1wt cells were grown in DMEM supplemented with 10% fetal bovine serum (FBS), BxPC3 and Capan1 cells were grown in RPMI supplemented with 10% fetal bovine serum (FBS) and CFPAC cells were grown in IMDM supplemented with 10% fetal bovine serum (FBS). All were incubated at 37°C in a 5% CO2-95% air atmosphere.

Cells were cultured two days before at the concentration of 1*10⁶ per 10 cm plate then the supernatant was discarded and the plate rinsed with 5 mL of PBS and trypsinated. The cells were then resuspended in 100 µL of PBA (PBS+2% FCS). Cells were then plated at the concentration of 1*10⁵ cells /point.

At this point, the work was performed at 4°C. Cells were centrifuged for 3 minutes at 1200 rpm and resuspended in 100 µL of a solution comprising a RBD ligand **(Table 1).** The RBD ligand is incubated with the cells for 30 minutes at 37°C (some RBD can also function *in vitro* at lower temperatures, down to 4°C). The cells were then centrifuged and rinsed with PBA, then resuspended in 100 µL anti-mouse A647 or anti-rabbit A647 (1/500 in PBA) for flow cytometry analysis.

**Table 1: lists of the RBD ligand used for the in vitro experiments.**

| **RBD** | **Transporter** |
|---|---|
| H2.RBD.GFP (SEQ ID NO: 32 fused to GFP) | GLUT-1 |
| XENO.RBD.RFC (SEQ ID NO: 8) | XPR1 |
| HERV-W.RBD.MFC (SEQ ID NO: 7) | ASCT-1/ASCT-2 |

### Results

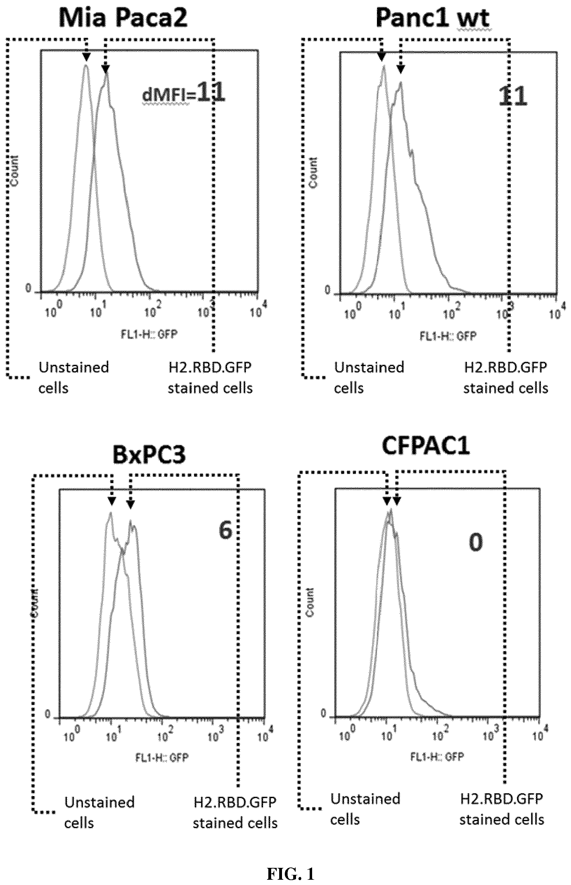

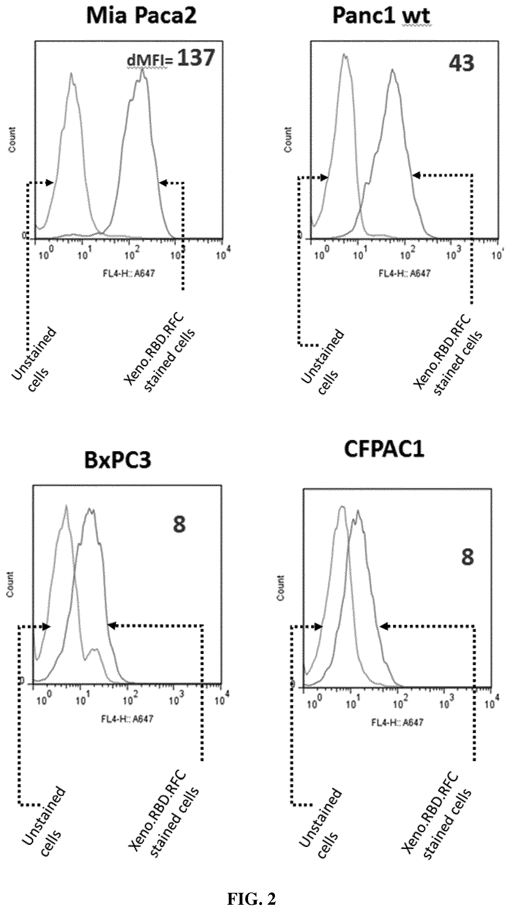

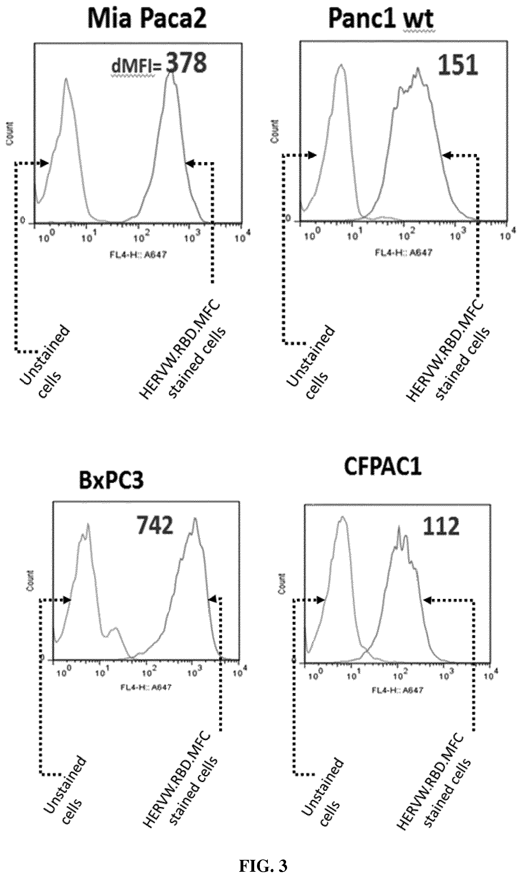

We tested the capacity of RBD ligands of the invention to specifically recognize pancreatic cell lines *in vitro.* As shown in **Figures 2** and **3****,** Xeno.RBD and HERV-W.RBD efficiently detect the pancreatic cell lines MiaPaca2, Panc1wt, BxPC3 and CFPAC. RBD ligands of the invention also specifically detect the Capan1 cell line (data not shown). On the contrary, H2.RBD does not allow the detection of these cell lines *in vitro* **(****Figure 1****).**

### Example 2

### Materials and Methods

### Radiolabeling of the RBD ligand

I-125 was obtained from Perkin Elmer, and RBDs ligands were radiolabeled at the specific activity of 370 MBq/mg for SPECT imaging, using the IODO-GEN (Pierce Chemical Co.) method as previously described (Santoro etal. 2009. J. Nucl. Med. 50:2033-2041). The marked ligand used for the *in vivo* study are HERV-W.RBD.MFC (SEQ ID NO: 7); Xeno.RBD.RFC (SEQ ID NO: 8); and H2.RBD.GFP (SEQ ID NO: 32) with I-125 (10 µCi/µg).

### Animals

All animal experiments were performed in compliance with the guidelines of the French government and the standards of Institut National de la Santé et de la Recherche Médicale for experimental animal studies (agreement D34-172-27).

Mice (5-week-old athymic FoxN1 mice) were obtained from Charles River/Harlan Laboratories and were acclimated for 1 week before experimental use. They were housed at 22°C and 55% humidity with a light-dark cycle of 12 hours. Food and water were available *ad libitum.* The mice were force-fed with Lugol solution the day before imaging, and stable iodine was added to drinking water for the entire experimental period.
- Group 1 (6 mice): left side: CFPAC (injection of 5.10⁶ cells) / right side: HPAC (injection of 5.10⁶ cells);
- Group 2 (6 mice): left side: CFPAC (injection of 5.10⁶ cells);
- Group 3 (6 mice): control group.

RBD is injected intravenously at 50 µg/mouse (radioactivity injected = 500 µCi/mouse).

During acquisitions mice were under anesthetic gas isoflurane 2.5%.

### SPECT-CT imaging

Whole-body SPECT/CT images were acquired at various times after tail vein injection of 18 MBq radiolabeled I-125.RBD. Mice were anesthetized with 2% isoflurane and positioned on the bed of 4-head multiplexing multipinhole NanoSPECT camera (Bioscan Inc., Washington, USA).

Energy window was centered at 28 keV with ±20% width, acquisition times were defined to obtain 30 000 counts for each projection with 24 projections. Images and maximum intensity projections (MIPs) were reconstructed using the dedicated software Invivoscope^{®} (Bioscan, Inc., Washington,USA) and Mediso InterViewXP^{®} (Mediso, Budapest Hungary). Concurrent microCT whole-body images were performed for anatomic co-registration with SPECT data. Reconstructed data from SPECT and CT were visualized and co-registered using Invivoscope^{®}.

Acquisitions with NanoSPECT-CT (Mediso):
SPECT-CT imaging was carried out 5 weeks post graft;
- 4h, 24h, 48h, 72h after injection of I-125.H2.RBD.GFP;
- 24h, 48h, 72h, 96h after injection of I-125.HERV-W.RBD.MFC and I-125.Xeno.RBD.RFC.

Image analysis was led with VivoQuant software and measures done counts/mm³.

### Results

The animals were selected with homogeneous tumors; however, depending on the tumor cell type, the tumors did not grow at the same speed and therefore do not have the same volume between each group of animals and between tumor cell types at imaging.

To overcome the different tumor volumes in image analysis, the measured values correspond to the radioactivity measured per unit volume of tumor tissue and is expressed as counts/mm³ of tumor volume. Thus, a comparison of the radioactivity associated with RBD is possible, according to various tumor cell types without having a bias induced by the different volumes of the tumors.

The measured values for the control animals correspond to the radioactivity per unit volume in the whole body of the animal (counts/mm3). Areas not taken into account in all animals for analysis are the bladder due to the natural elimination (this may vary over time depending on the animal) and the tail as it is the injection site (residues can persist). The noise background is the natural and surrounding radioactivity detectors that can take into account independently of a radioactive source. The values measured in the range of the noise background are not significant.

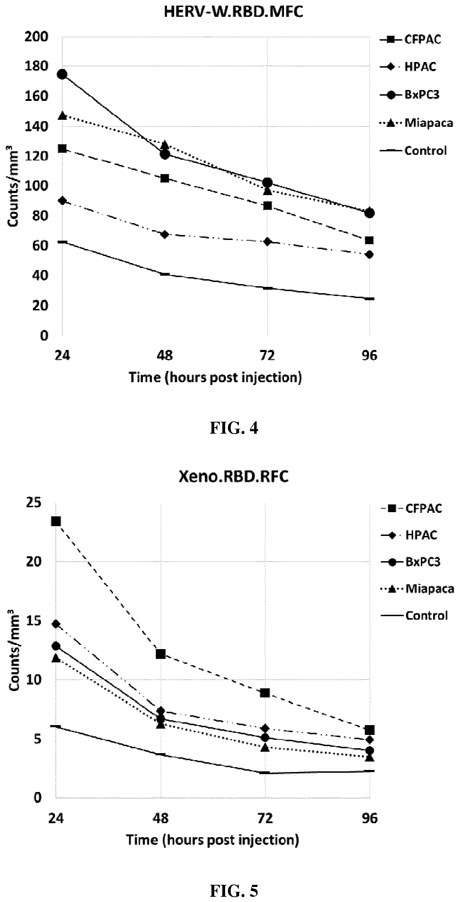

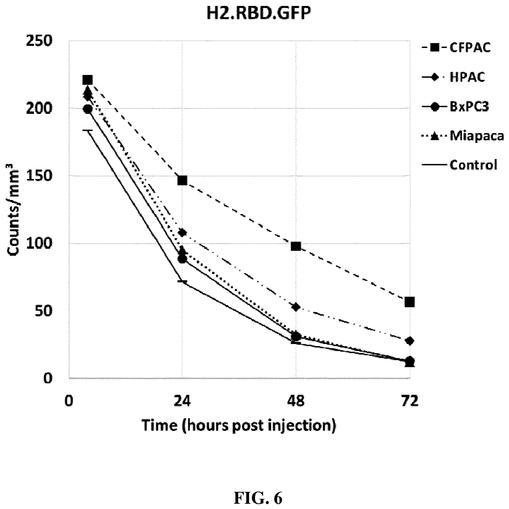

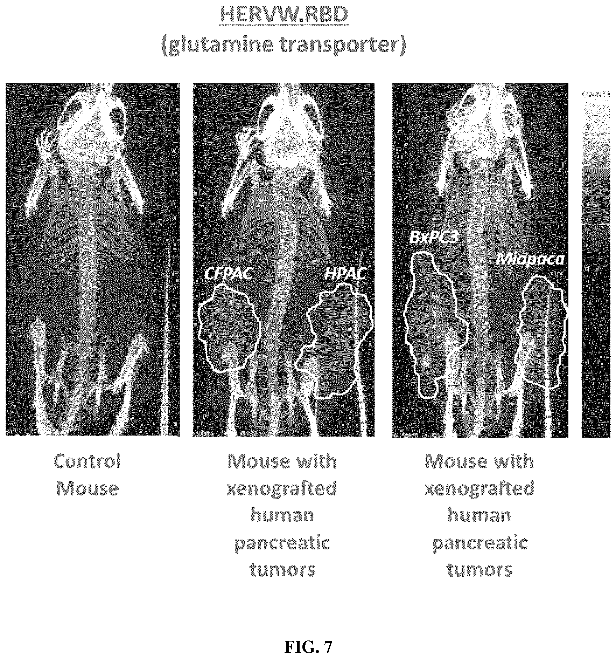

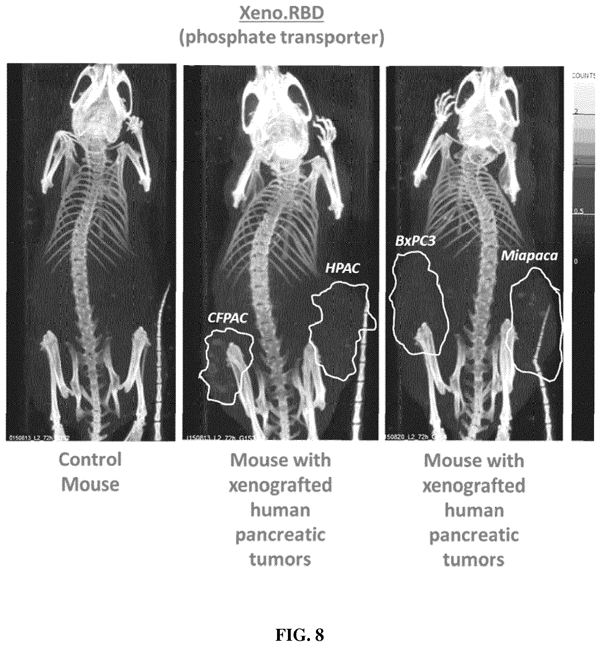

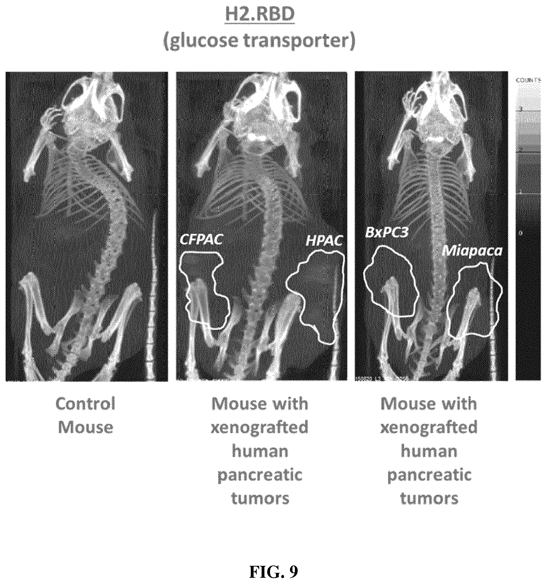

**Figures 4-5** and **7-8** demonstrate the recognition *in vivo* of different kind of pancreatic tumor cells by I-125.HERV-W.RBD.MFC and I-125.Xeno.RBD.RFC respectively. On the contrary, as demonstrated by **Figures 6** and **9****,** I-125.H2.RBD.GFP does not allow efficient recognition of pancreatic cancer cells *in vivo.*

## Claims

1. A ligand comprising a part or the totality of a receptor-binding domain (RBD) derived from the soluble part of a glycoprotein of an enveloped virus for use for the *in vivo* detection of pancreatic cancer cells in a patient, wherein said ligand is specific of a cell surface nutrient transporter selected from ASCT1, ASCT2 and/or XPR1.

2. The ligand for use according to claim 1, for the diagnosis or monitoring of pancreatic cancer.

3. The ligand for use according to claim **1** or **2,** for the diagnosis or monitoring of pancreatic cancer preferably by medical imaging, preferably by magnetic resonance imaging (MRI), X-ray-based imaging techniques such as computed tomography (CT), radiography, positron-emission tomography (PET), single photon emission tomography (SPECT), endoscopic ultrasound (EUS), magnetic resonance cholangiopancreatography, fluorimetry, fluoroscopy, fluorescence, and near-infrared (NIR) fluorescent imaging.

4. The ligand for use according to any one of claims **1** to **3,** wherein said ligand is selected from the group comprising HERV-W.RBD, RD114.RBD, BaEV.RBD, SNV.RBD, SRV.RBD, MPMV.RBD, Xeno.RBD, variants and fragments thereof.

5. The ligand for use according to any one of claims **1** to **4,** wherein said ligand is coupled with at least one contrast agent, wherein said contrast agent is preferably selected from a radiolabeled agent or a fluorescent agent.

6. An *in vitro* method for detecting pancreatic cancer cells, wherein said method comprises measuring the expression level of at least one cell surface nutrient transporter selected from ASCT1, ASCT2 and/or XPR1, wherein said expression level is assessed at the protein level, by detecting and/or quantifying binding of a ligand to a cell surface nutrient transporter, wherein said ligand comprises a part or the totality of a RBD derived from the soluble part of a glycoprotein of an enveloped virus.

7. The *in vitro* method according to claim **6,** wherein said method further comprises comparing the measured expression level with a reference expression level.

8. The *in vitro* method according to claim **6** or **7,** wherein said method is for diagnosing or monitoring pancreatic cancer in a subject.

9. The *in vitro* method according to any one of claims **6** to **8,** wherein said ligand is HERV-W.RBD, RD114.RBD, BaEV.RBD, SNV.RBD, SRV.RBD, MPMV.RBD, or Xeno.RBD, variants or fragments thereof.

10. A diagnostic composition for the in vivo detection of pancreatic cancer cells in a patient, said composition comprising at least one ligand and a pharmaceutically acceptable excipient, said ligand comprising a part or the totality of a receptor-binding domain (RBD) derived from the soluble part of a glycoprotein of an enveloped virus, wherein said ligand is specific of a cell surface nutrient transporter selected from ASCT1, ASCT2 and/or XPR1

11. A kit for the in vivo detection of pancreatic cancer cells in a patient, said kit
comprising at least one ligand comprising a part or the totality of a receptor-binding domain (RBD) derived from the soluble part of a glycoprotein of an enveloped virus, wherein said ligand is specific of a cell surface nutrient transporter selected from ASCT1, ASCT2 and/or XPR1.

12. A pharmaceutical composition for use for the treatment of pancreatic cancer comprising at least one RBD ligand selected from the group comprising HERV-W.RBD, RD114.RBD, BaEV.RBD, SNV.RBD, SRV.RBD, MPMV.RBD, Xeno.RBD, variants and fragments thereof, and a pharmaceutically acceptable excipient.

## Patentansprüche

1. Ligand, umfassend einen Teil oder die Gesamtheit einer rezeptorbindenden Domäne (RBD), die von dem löslichen Teil eines Glykoproteins eines behüllten Virus abgeleitet ist, zur Verwendung zum *In-vivo*-Nachweis von Bauchspeicheldrüsenkrebszellen in einem Patienten, wobei der Ligand für einen Zelloberflächennährstofftransporter spezifisch ist, der aus ASCT1, ASCT2 und/oder XPR1 ausgewählt ist.

2. Ligand zur Verwendung nach Anspruch 1, zur Diagnose oder Überwachung von Bauchspeicheldrüsenkrebs.

3. Ligand zur Verwendung nach Anspruch **1** oder **2,** zur Diagnose oder Überwachung von Bauchspeicheldrüsenkrebs, vorzugsweise durch medizinische Bildgebung, vorzugsweise durch Magnetresonanztomographie (MRT), Bildgebungstechniken auf Röntgenbasis, wie Computertomographie (CT), Röntgenografie, Positronenemissionstomographie (PET), Einzelphotonenemissionstomographie (SPECT), endoskopischer Ultraschall (EUS), Magnetresonanzcholangiopankreatographie, Fluorimetrie, Fluoroskopie, Fluoreszenz- und Nahinfrarot-Fluoreszenzbildgebung (NIR-Fluoreszenzbildgebung).

4. Ligand zur Verwendung nach einem der Ansprüche **1** bis **3,** wobei der Ligand aus der Gruppe umfassend HERV-W.RBD, RD114.RBD, BaEV.RBD, SNV.RBD, SRV.RBD, MPMV.RBD, Xeno.RBD, Varianten und Fragmenten davon ausgewählt ist.

5. Ligand zur Verwendung nach einem der Ansprüche **1** bis **4,** wobei der Ligand mit mindestens einem Kontrastmittel gekoppelt wird, wobei das Kontrastmittel vorzugsweise aus einem radiomarkierten Mittel oder einem fluoreszierenden Mittel ausgewählt ist.

6. *In-vitro*-Verfahren zum Nachweisen von Bauchspeicheldrüsenkrebszellen, wobei das Verfahren ein Messen des Expressionsniveaus von mindestens einem Zelloberflächennährstofftransporter, der aus ASCT1, ASCT2 und/oder XPR1 ausgewählt ist, umfasst, wobei das Expressionsniveau auf der Proteinebene durch Nachweisen und/oder Quantifizieren einer Bindung eines Liganden an einen Zelloberflächennährstofftransporter beurteilt wird, wobei der Ligand einen Teil oder die Gesamtheit einer RBD, die von dem löslichen Teil eines Glykoproteins eines behüllten Virus abgeleitet ist, umfasst.

7. *In-vitro*-Verfahren nach Anspruch **6,** wobei das Verfahren weiterhin ein Vergleichen des gemessenen Expressionsniveaus mit einem Referenzexpressionsniveau umfasst.

8. *In-vitro*-Verfahren nach Anspruch **6** oder **7,** wobei das Verfahren zur Diagnose oder Überwachung von Bauchspeicheldrüsenkrebs bei einem Probanden ist.

9. *In-vitro*-Verfahren nach einem der Ansprüche 6 bis 8, wobei es sich bei dem Liganden um HERV-W.RBD, RD114.RBD, BaEV.RBD, SNV.RBD, SRV.RBD, MPMV.RBD oder Xeno.RBD, Varianten oder Fragmente davon handelt.

10. Diagnostische Zusammensetzung zum In-vivo-Nachweis von Bauchspeicheldrüsenkrebszellen in einem Patienten, wobei die Zusammensetzung mindestens einen Liganden und einen pharmazeutisch annehmbaren Hilfsstoff umfasst, wobei der Ligand einen Teil oder die Gesamtheit einer rezeptorbindenden Domäne (RBD), die von dem löslichen Teil eines Glykoproteins eines behüllten Virus abgeleitet ist, umfasst, wobei der Ligand für einen Zelloberflächennährstofftransporter spezifisch ist, der aus ASCT1, ASCT2 und/oder XPR1 ausgewählt ist.

11. Kit zum In-vivo-Nachweis von Bauchspeicheldrüsenkrebszellen in einem Patienten, wobei das Kit mindestens einen Liganden umfasst, der einen Teil oder die Gesamtheit einer rezeptorbindenden Domäne (RBD), die von dem löslichen Teil eines Glykoproteins eines behüllten Virus abgeleitet ist, umfasst, wobei der Ligand für einen Zelloberflächennährstofftransporter spezifisch ist, der aus ASCT1, ASCT2 und/oder XPR1 ausgewählt ist.

12. Pharmazeutische Zusammensetzung zur Verwendung zur Behandlung von Bauchspeicheldrüsenkrebs, umfassend mindestens einen RBD-Liganden, der aus der Gruppe umfassend HERV-W.RBD, RD114.RBD, BaEV.RBD, SNV.RBD, SRV.RBD, MPMV.RBD, Xeno.RBD, Varianten und Fragmente davon ausgewählt ist, und einen pharmazeutisch annehmbaren Hilfsstoff.

## Revendications

1. Un ligand comprenant une partie ou la totalité d'un domaine de liaison au récepteur (RBD) dérivé de la partie soluble d'une glycoprotéine d'un virus enveloppé pour son utilisation pour la détection *in vivo* de cellules cancéreuses du pancréas chez un patient, dans lequel ledit ligand est spécifique d'un transporteur de nutriment à la surface cellulaire sélectionné parmi ASCT1, ASCT2 et/ou XPR1.

2. Le ligand pour son utilisation selon la revendication **1,** pour le diagnostic ou la surveillance d'un cancer du pancréas.

3. Le ligand pour son utilisation selon la revendication **1** ou **2,** pour le diagnostic ou la surveillance d'un cancer du pancréas par imagerie médicale, de préférence par imagerie par résonance magnétique (IRM), techniques d'imagerie basées sur les rayons X telles que tomodensitométrie (CT), radiographie, tomographie par émission de positrons (PET), tomographie par émission de photons uniques (SPECT), échographie endoscopique (EUS), cholangiopancréatographie par résonance magnétique, fluorimétrie, fluoroscopie, fluorescence, et imagerie de fluorescence proche infrarouge (NIR).

4. Le ligand pour son utilisation selon l'une quelconque des revendications **1** à **3,** dans lequel ledit ligand est sélectionné parmi le groupe comprenant HERV-W.RBD, RD114.RBD, BaEV.RBD, SNV.RBD, SRV.RBD, MPMV.RBD, Xeno.RBD, des variants et fragments de ceux-ci.

5. Le ligand pour son utilisation selon l'une quelconque des revendications **1** à **4,** dans lequel ledit ligand est couplé avec au moins un agent de contraste, dans lequel ledit agent de contraste est de préférence sélectionné parmi un agent radiomarqué ou un agent fluorescent.

6. Une méthode *in vitro* pour détecter des cellules de cancer du pancréas, dans laquelle ladite méthode comprend la mesure du niveau d'expression d'au moins un transporteur de nutriment à la surface cellulaire sélectionné parmi ASCT1, ASCT2 et/ou XPR1, dans laquelle ledit niveau d'expression est évalué au niveau protéique, en détectant et/ou quantifiant la liaison d'un ligand à un transporteur de nutriment à la surface cellulaire, dans laquelle ledit ligand comprend une partie ou la totalité d'un RBD dérivé de la partie soluble d'une glycoprotéine d'un virus enveloppé.

7. La méthode *in vitro* selon la revendication **6,** dans laquelle ladite méthode comprend en outre la comparaison du niveau d'expression mesuré avec un niveau d'expression de référence.

8. La méthode *in vitro* selon la revendication **6** ou **7,** dans laquelle ladite méthode est pour diagnostiquer ou surveiller un cancer du pancréas chez un sujet.

9. La méthode *in vitro* selon l'une quelconque des revendications **6** à **8,** dans laquelle ledit ligand est HERV-W.RBD, RD 1 14.RBD, BaEV.RBD, SNV.RBD, SRV.RBD, MPMV.RBD, ou Xeno.RBD, des variants et fragments de ceux-ci.

10. Une composition diagnostic pour la détection *in vivo* de cellules de cancer du pancréas chez un patient, ladite composition comprenant au moins un ligand et un excipient pharmaceutiquement acceptable, ledit ligand comprenant une partie ou la totalité d'un domaine de liaison au récepteur (RBD) dérivé de la partie soluble d'une glycoprotéine d'un virus enveloppé, dans laquelle ledit ligand est spécifique d'un transporteur de nutriment à la surface cellulaire sélectionné parmi ASCT1, ASCT2 et/ou XPR1.

11. Un kit pour la détection *in vivo* de cellules de cancer du pancréas chez un patient, ledit kit comprenant au moins un ligand comprenant une partie ou la totalité d'un domaine de liaison au récepteur (RBD) dérivé de la partie soluble d'une glycoprotéine d'un virus enveloppé, dans lequel ledit ligand est spécifique d'un transporteur de nutriment à la surface cellulaire sélectionné parmi ASCT1, ASCT2 et/ou XPR1.

12. Une composition pharmaceutique pour son utilisation pour le traitement d'un cancer du pancréas comprenant au moins un ligand RBD sélectionné parmi le groupe comprenant HERV-W.RBD, RD114.RBD, BaEV.RBD, SNV.RBD, SRV.RBD, MPMV.RBD, Xeno.RBD, des variants et fragments de ceux-ci, et un excipient pharmaceutiquement acceptable.
